(19) Europäisches Patentamt
European Patent Office
Office européen des brevets

(11) Publication number: **0 105 735**
**A2**

(12) # EUROPEAN PATENT APPLICATION

(21) Application number: 83305909.0

(22) Date of filing: 29.09.83

(51) Int. Cl.³: C 07 C 127/19
C 07 C 149/437, A 01 N 47/30
C 07 D 521/00
//C07D215/32, C07D213/75

(30) Priority: 30.09.82 US 430368

(43) Date of publication of application:
18.04.84 Bulletin 84/16

(84) Designated Contracting States:
AT BE CH DE FR GB IT LI LU NL SE

(71) Applicant: UNION CARBIDE CORPORATION
Old Ridgebury Road
Danbury Connecticut 06817(US)

(72) Inventor: Wheeler, Thomas Neil
8605 Woodlawn Drive
Raleigh North Carolina 27612(US)

(74) Representative: Baverstock, Michael George Douglas et al,
BOULT, WADE & TENNANT 27 Furnival Street
London, EC4A 1PQ(GB)

(54) **Herbicidal ureas and process for preparation.**

(57) Urea compounds are provided which exhibit primarily post-emergence control of broadleaf weeds and which also show a remarkable degree of selectivity for soybeans, corn, wheat and other crops.

The compounds have the general formula:

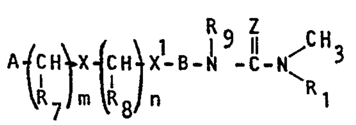

wherein:

A is

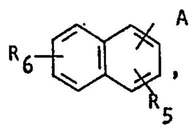, 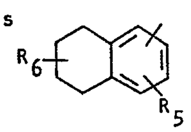, 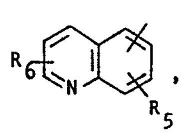, 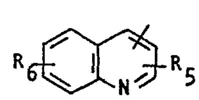,

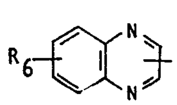, 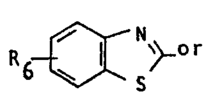 or 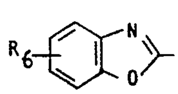

B is

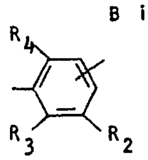 or 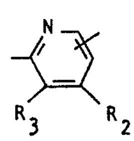

./...

EP 0 105 735 A2

X and X' are independently oxygen, sulfur, or a carbon-carbon, carbon-oxygen, or a carbon-sulfur single bond with the proviso that X and X' may not both be a carbon-carbon single bond at the same time;

Z is oxygen or sulfur;

$R_1$ is hydrogen, $C_1$-$C_8$ alkyl, or $C_1$-$C_3$ alkoxy;

$R_2$-$R_6$ are independently hydrogen, $C_1$-$C_{10}$ alkyl, halogen, cyano, nitro, trifluoromethyl, $C_1$-$C_5$ alkoxyl, or substituted phenoxy; $R_6$ may also be keto when attached to a saturated ring;

$R_7$-$R_9$ are independently hydrogen or lower ($C_1$-$C_3$) alkyl; and

m and n represent the same or different integers in the range 0 to 10 with the proviso that m and n may not both be zero when A is 2-napthyl.

# HERBICIDAL UREAS AND PROCESS FOR PREPARATION

This invention relates in general to ureas compounds and to a process for their preparation and use. In one aspect, this invention is directed to ureas which exhibit primarily post-emergence control of broadleaf weeds. In a further aspect, this invention relates to herbicides which show a remarkable degree of selectivity for soybeans, corn, wheat, and other crops.

Prior to the present invention, several substituted phenyl ureas were known which exhibited herbicidal activity. For example, phenyl ureas, such as monuron (N'-4-chlorophenyl-N,N-dimethyl urea) and diuron (N'-3,4-dichlorophenyl-N,N-dimethylurea) possess a pronounced herbicidal activity that is attributed to inhibition of photosynthesis. However, although quite active, these ureas show a broad spectrum of herbicidal activity and damage important crops, e.g. corn and soybeans. Other ureas are described in the patent literature, see, for example, U.S. - A - 4,280,835; 4,249,938; 4,289,903, 4,129,436; 4,263,219, 4,309,212, and 4,260,411. However, while many of the compounds disclosed in these patents are indicated to possess some herbicidal activity, there does not appear to be any common link from which one could predict the activity of other phenyl ureas. Moreover, even those compounds which are disclosed to be active, are usually so broad in the scope of their activity that they can

not be used for the control of broadleaf weeds without the desired crop also sustaining injury.

Hence, a major advantage shown by the compounds of the present invention is that while they retain the high level of herbicidal activity typical of certain phenyl ureas, they simultaneously exhibit good selectivity for, and can be safely applied to, many important crops such as soybeans, corn, and grains.

In its broad aspect, the invention relates to ureas, herbicidal compositions containing the same, and processes for their preparation and use. The ureas of this invention are represented by the following generic formula:

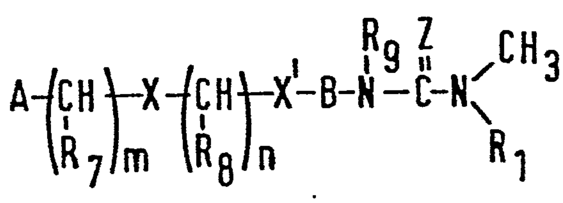

1

wherein the various substituents are identified below.

As previously indicated, this invention is directed to the synthesis of ureas which exhibit excellent postemergence control of broadleaf weeds and which show a remarkable degree of selectivity for soybeans, corn, wheat and certain other crops.

The ureas of this invention are defined by the generic formula:

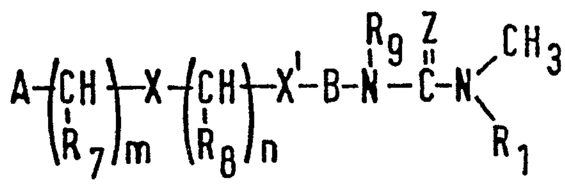

where A is:

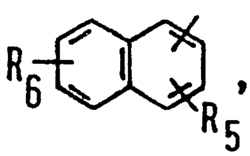, 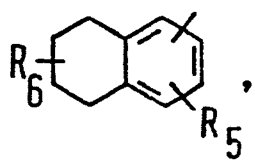, 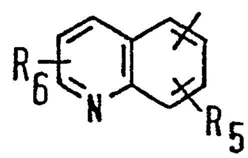, 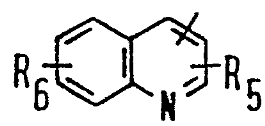,

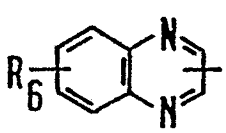, 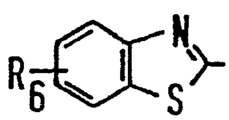 or 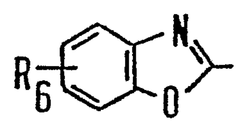

where B is:

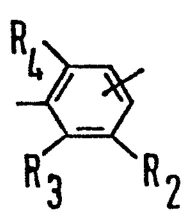 or 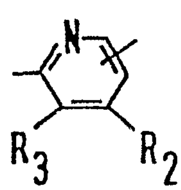

X and X' are independently oxygen, sulfur,

or a carbon-carbon, carbon-oxygen, or carbon-sulfur single bond with the proviso that X and X' may not both be a carbon-carbon single bond at the same time;

Z is oxygen or sulfur;

$R_1$ is hydrogen; $C_1$-$C_8$ alkyl, or $C_1$-$C_3$ alkoxy;

$R_2$-$R_6$ are independently hydrogen, $C_1$-$C_{10}$ alkyl, halogen, cyano, nitro, trifluoromethyl, $C_1$-$C_5$ alkoxy, or substituted phenoxy; $R_6$ may also be keto when attached to a saturated ring;

$R_7$-$R_9$ are independently hydrogen or lower ($C_1$-$C_3$) alkyl;

m and n represent the same or different integers in the range of 0 to 10 with the proviso that A may not be 2-naphthoxy when m=n=0.

Illustrative urea compounds which can be prepared in accordance with the teachings of this invention include, but are not limited to:

1-methyl-1-methoxy-3-[4-[2-(1-naphth)]ethyloxyphenyl] urea,

1,1-dimethyl-3-[4-[2-(1-naphth)]ethyloxyphenyl]urea,

1,1-methyl-1-methoxy-3-[4-[2-(1-naphth)]ethyloxy-3-chlorophenyl]urea,

1,1-dimethyl-3-[4-[2-(1-naphth)]ethyloxy-3-chlorophenyl]urea,

1-methyl-1-methoxy-3-[4-[2-(1-naphth)]ethyloxy-3-trifluoromethylphenyl]urea,

1,1-dimethyl-3-[4-[2-(1-naphth)]ethyloxy-3-trifluoromethylphenyl]urea,

1-methyl-1-methoxy-3-[4-(5,6,7,8-tetrahydro-1-naphthoxy)phenyl]urea,

1,1-dimethyl-3-[4-(5,6,7,8-tetrahydro-1-naphthoxy)-phenyl]urea,
1-methyl-1-methoxy-3-[4-(5,6,7,8-tetrahydro-1-naphthoxy)-3-chlorophenyl]urea,
1,1-dimethyl-3-[4-(5,6,7,8-tetrahydro-1-naphthoxy)-3-chlorophenyl]urea,
1-methyl-1-methoxy-3-[4-(5,6,7,8-tetrahydro-1-naphthoxy)-3-trifluoromethylphenyl]-urea.
1,1-dimethyl-3-[4-(5,6,7,8-tetrahydro-1-naphthoxy)-3-trifluoromethylphenyl]urea.

It is accordingly, readily apparent from the scope of the preceding formulae that this invention encompasses a wide variety of ureas. Tables A-M which follow, set forth sub classes within the broad generic formula and illustrate specific compounds falling within the particular classes. These examples will serve to illustrate but not limit the areas defined by this invention.

TABLE A

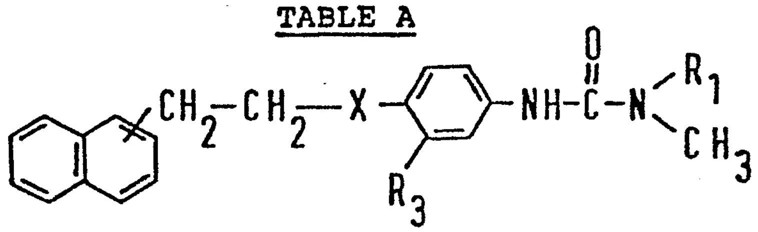

| Ring Position on Napthyl | X | $R_3$ | $R_1$ |
|---|---|---|---|
| 1 | O | H | $CH_3$ |
| 1 | O | H | $OCH_3$ |
| 2 | O | H | $CH_3$ |
| 2 | O | H | $OCH_3$ |
| 1 | S | H | $CH_3$ |
| 1 | S | H | $OCH_3$ |
| 1 | O | $CH_3$ | $CH_3$ |
| 1 | O | $CH_3$ | $OCH_3$ |
| 1 | O | Cl | $CH_3$ |
| 1 | O | Cl | $OCH_3$ |
| 1 | O | $CF_3$ | $CH_3$ |
| 1 | O | $CF_3$ | $OCH_3$ |
| 1 | O | $NO_2$ | $CH_3$ |
| 1 | O | $NO_2$ | $OCH_3$ |
| 1 | O | CN | $CH_3$ |

| | | | |
|---|---|---|---|
| 1 | O | CN | $OCH_3$ |
| 1 | O | $OCH_3$ | $CH_3$ |
| 1 | O | $OCH_3$ | $OCH_3$ |
| 1 | S | Cl | $CH_3$ |
| 1 | S | Cl | $OCH_3$ |
| 1 | S | $CF_3$ | $CH_3$ |

TABLE B

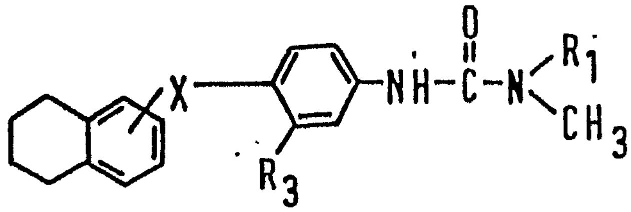

| Tetrahydronaphthyl Ring Position | X | $R_3$ | $R_1$ |
|---|---|---|---|
| 1 | O | H | $CH_3$ |
| 1 | O | H | $OCH_3$ |
| 2 | O | H | $CH_3$ |
| 2 | O | H | $OCH_3$ |
| 1 | O | Cl | $CH_3$ |
| 1 | O | Cl | $OCH_3$ |
| 2 | O | Cl | $CH_3$ |
| 2 | O | Cl | $OCH_3$ |
| 1 | O | $CF_3$ | $CH_3$ |
| 1 | O | $CF_3$ | $OCH_3$ |
| 2 | O | $CF_3$ | $CH_3$ |
| 2 | O | $CF_3$ | $OCH_3$ |
| 1 | O | $CH_3$ | $CH_3$ |
| 1 | O | $CH_3$ | $OCH_3$ |
| 2 | O | $CH_3$ | $CH_3$ |
| 2 | O | $CH_3$ | $OCH_3$ |
| 1 | O | $NO_2$ | $CH_3$ |
| 1 | O | $NO_2$ | $OCH_3$ |
| 1 | O | CN | $CH_3$ |
| 1 | O | CN | $OCH_3$ |
| 1 | O | $OCH_3$ | $OCH_3$ |
| 1 | O | $OCH_3$ | $CH_3$ |
| 1 | S | H | $CH_3$ |
| 1 | S | H | $OCH_3$ |
| 2 | S | H | $CH_3$ |
| 2 | S | H | $OCH_3$ |
| 1 | S | Cl | $OCH_3$ |
| 1 | S | $CF_3$ | $OCH_3$ |
| 1 | S | $CH_3$ | $OCH_3$ |

TABLE C

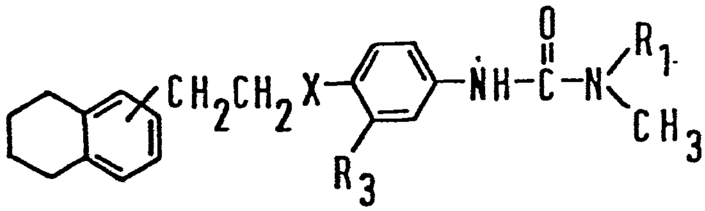

| Tetrahydronaphthyl Ring Position | X | $R_3$ | $R_1$ |
|---|---|---|---|
| 1 | O | H | $CH_3$ |
| 1 | O | H | $OCH_3$ |
| 2 | O | H | $CH_3$ |
| 2 | O | H | $OCH_3$ |
| 1 | O | Cl | $CH_3$ |
| 1 | O | Cl | $OCH_3$ |
| 2 | O | Cl | $CH_3$ |
| 2 | O | Cl | $OCH_3$ |
| 1 | O | $CF_3$ | $OCH_3$ |
| 1 | O | $NO_2$ | $OCH_3$ |
| 1 | O | $CH_3$ | $OCH_3$ |
| 1 | S | H | $CH_3$ |
| 1 | S | H | $OCH_3$ |
| 2 | S | H | $CH_3$ |
| 2 | S | H | $OCH_3$ |
| 1 | S | Cl | $CH_3$ |
| 2 | S | Cl | $CH_3$ |
| 1 | S | $CF_3$ | $OCH_3$ |
| 2 | S | $CF_3$ | $OCH_3$ |
| 1 | O | $OCH_3$ | $CH_3$ |
| 1 | O | $OCH_3$ | $OCH_3$ |
| 1 | O | F | $OCH_3$ |
| 2 | O | F | $OCH_3$ |

## TABLE D

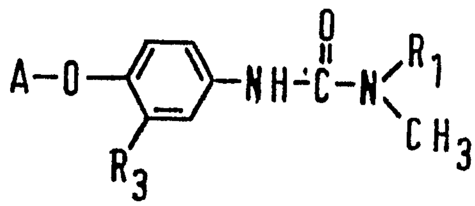

| A | $R_3$ | $R_1$ |
|---|---|---|
| 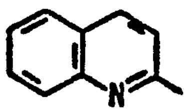 | H | $OCH_3$ |
| 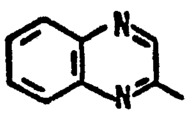 | H | $OCH_3$ |
| 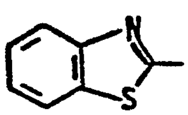 | H | $OCH_3$ |
| 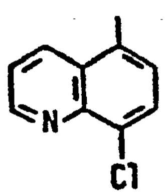 | H | $OCH_3$ |

TABLE E

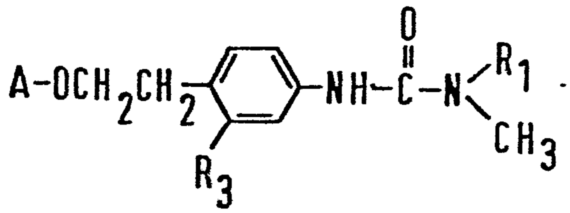

| A | $R_3$ | $R_1$ |
|---|---|---|
| 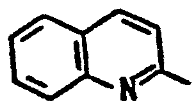 | H | $OCH_3$ |
| 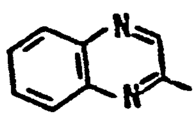 | H | $OCH_3$ |
| 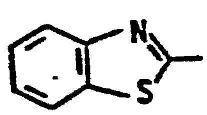 | H | $OCH_3$ |

TABLE F

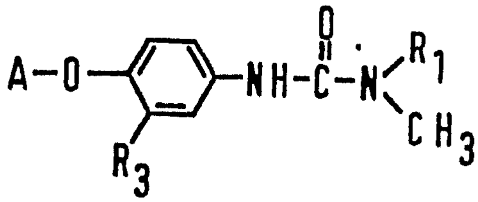

| A | $R_3$ | $R_1$ |
|---|---|---|
| 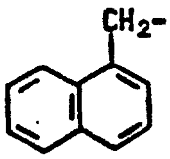 | H | $OCH_3$ |
| 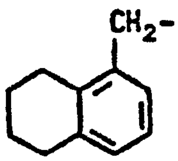 | H | $OCH_3$ |
| 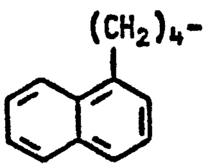 | H | $OCH_3$ |
| 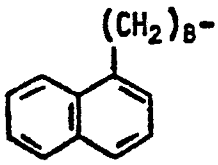 | H | $OCH_3$ |
| 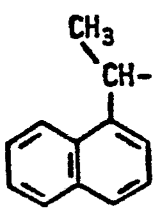 | H | $OCH_3$ |
| 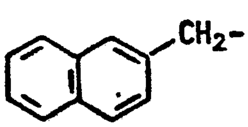 | H | $OCH_3$ |
| 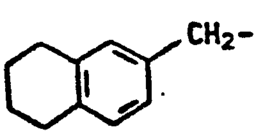 | H | $OCH_3$ |

TABLE G

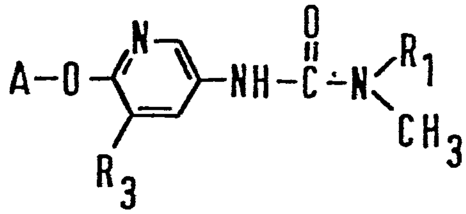

| A | $R_3$ | $R_1$ |
|---|---|---|
| 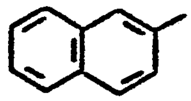 | H | $OCH_3$ |
| 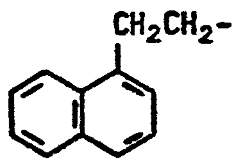 | H | $OCH_3$ |
| 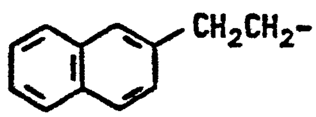 | H | $OCH_3$ |
| 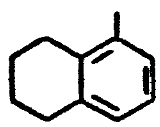 | H | $OCH_3$ |
| 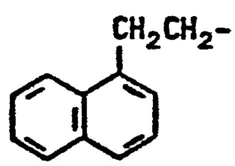 | $CH_3$ | $OCH_3$ |
| 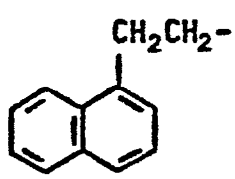 | Cl | $OCH_3$ |
| 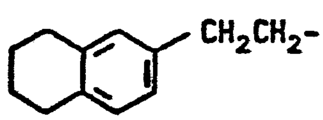 | H | $OCH_3$ |

TABLE H

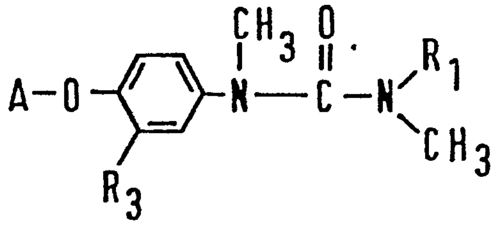

| A | $R_3$ | $R_1$ |
|---|---|---|
| 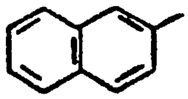 | H | H |
| 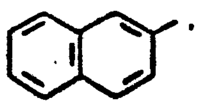 | H | $OCH_3$ |
| 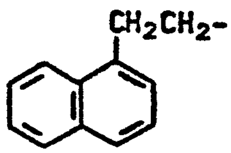 | H | H |
| 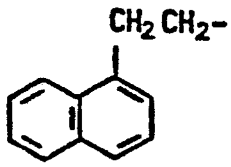 | H | $OCH_3$ |
| 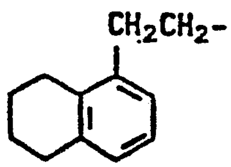 | H | H |
| 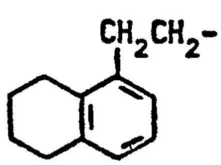 | H | $OCH_3$ |
| 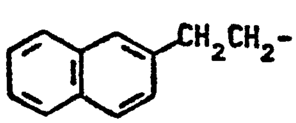 | H | $OCH_3$ |

TABLE I

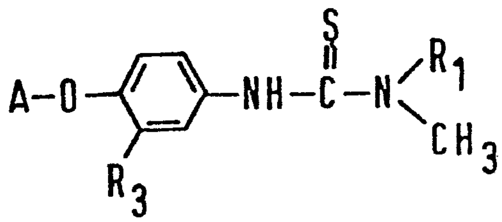

| A | $R_3$ | $R_1$ |
| --- | --- | --- |
| 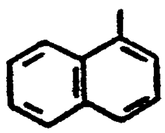 | H | $OCH_3$ |
| 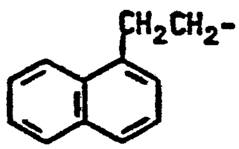 | H | $OCH_3$ |
| 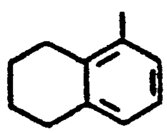 | H | $OCH_3$ |
| 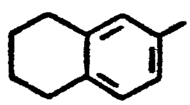 | H | $OCH_3$ |
| 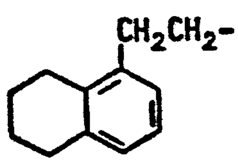 | H | $OCH_3$ |
| 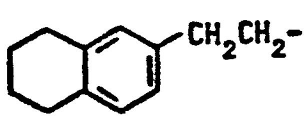 | H | $OCH_3$ |

TABLE J

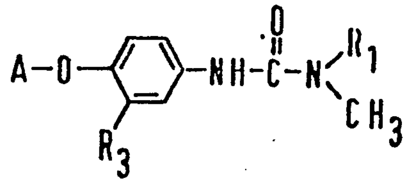

| A | $R_3$ | $R_1$ |
|---|---|---|
| 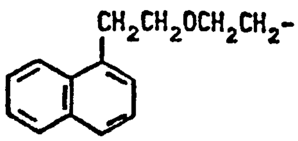 | H | $OCH_3$ |
| 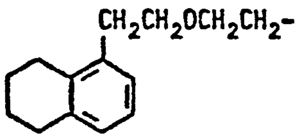 | H | $CH_3$ |
| 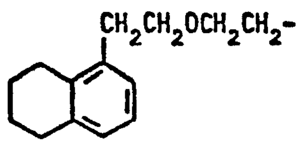 | H | $OCH_3$ |
| 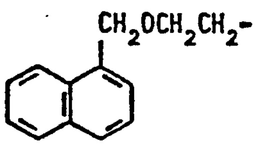 | H | $OCH_3$ |
| 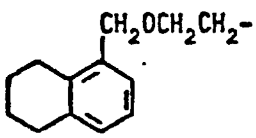 | H | $OCH_3$ |
| 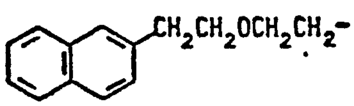 | H | $OCH_3$ |
| 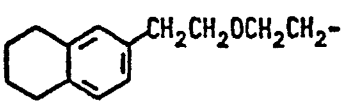 | H | $OCH_3$ |
| 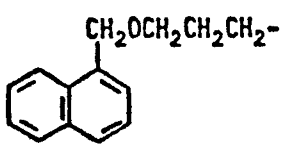 | H | $OCH_3$ |

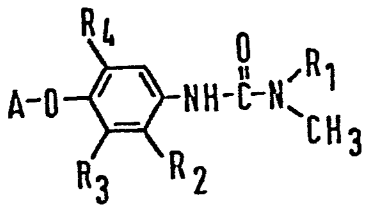

TABLE K

| A | $R_1$ | $R_2$ | $R_3$ | $R_4$ |
|---|---|---|---|---|
| 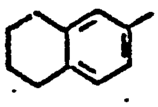 | $OCH_3$ | $CH_3$ | H | Cl |
| 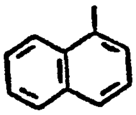 | $OCH_3$ | $CH_3$ | H | Cl |
| 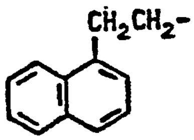 | $OCH_3$ | $CH_3$ | H | Cl |
| 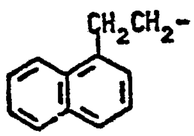 | $OCH_3$ | $CH_3$ | H | $CH_3$ |
| 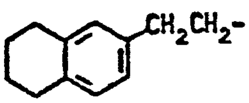 | $OCH_3$ | $CH_3$ | H | $CH_3$ |
| 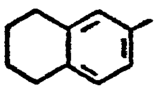 | $OCH_3$ | $CH_3$ | H | $CH_3$ |
| 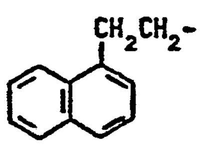 | $OCH_3$ | Cl | H | Cl |
| 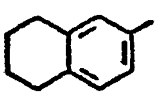 | $OCH_3$ | Cl | H | Cl |
| 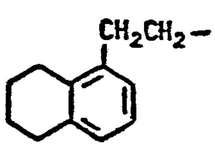 | $OCH_3$ | Cl | H | Cl |

TABLE L

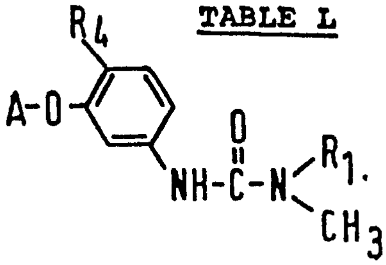

| A | $R_4$ | $R_1$ |
| --- | --- | --- |
| 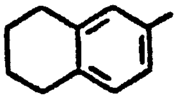 | H | $OCH_3$ |
| 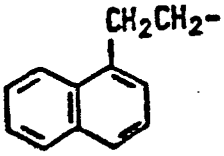 | H | $OCH_3$ |
| 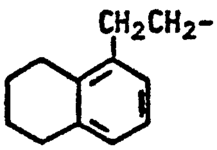 | H | $OCH_3$ |
| 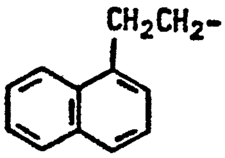 | Cl | $OCH_3$ |
| 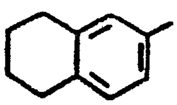 | Cl | $OCH_3$ |
| 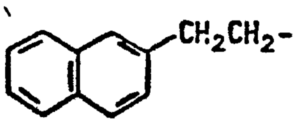 | Cl | $OCH_3$ |
| 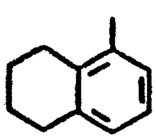 | Cl | $OCH_3$ |

TABLE M

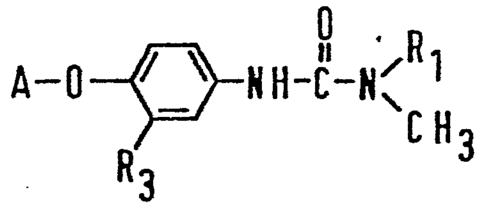

| A | $R_3$ | $R_1$ |
|---|---|---|
| 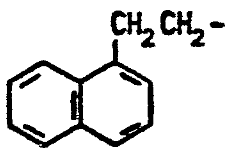 | H | H |
| 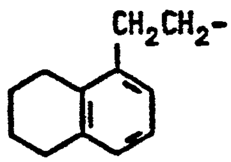 | H | H |
| 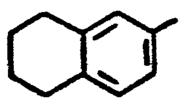 | H | $-CH_2CH_3$ |
| 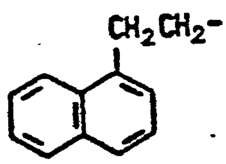 | H | $-OCH_2CH_3$ |
| 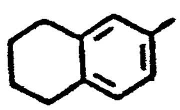 | H | $-OCH_2CH_3$ |
| 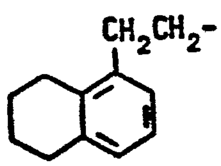 | H | $-OCH_2CH_3$ |

TABLE M (Cont'd)

| A | $R_3$ | $R_1$ |
|---|---|---|
| 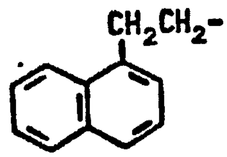 | H | $-OCH_2CH_2CH_3$ |
| 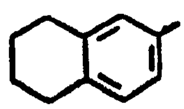 | H | $-(CH_2)_2CH_3$ |
| 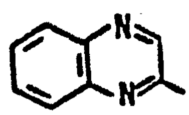 | H | $-OCH_2CH_3$ |
| 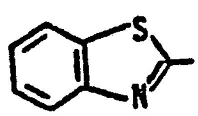 | H | $-CH_2CH_3$ |
| 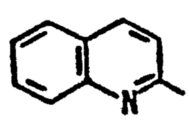 | H | $-OCH_2CH_3$ |

The urea compounds of this invention can be prepared by one or more routes as described below. For example, the compounds of formula 1 wherein m = n = 0 and $R_9$ = H, may be synthesized in the following manner:

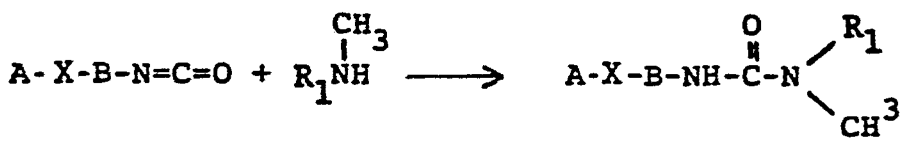

wherein A, X, B and $R_1$ are as previously defined. This reaction may be carried out in any of a variety of organic solvents (e.g., benzene, xylene, diethyl ether, tetrahydrofuran dioxane, N,N-dimethylformamide, chloroform, carbon tetrachloride) at a temperature of 0° to 50°C to give the desired ureas in very high yield.

The required aryl isocyanates are readily obtained by reaction of the corresponding aniline 7 with phosgene in an inert organic solvent.

$$\underset{7}{A\text{-}X\text{-}B\text{-}NH_2} + COCl_2 \longrightarrow A\text{-}X\text{-}B\text{-}N{=}C{=}O + HCl$$

The anilines 7 are obtained by reacting the appropriate napthols with activated aryl halides followed by reduction of the nitrophenyl ether with chemical methods or catalytic hydrogenation.

$$A\text{-}XH + L\text{-}B\text{-}NO_2 \longrightarrow A\text{-}X\text{-}B\text{-}NO_2 \longrightarrow A\text{-}X\text{-}B\text{-}NH_2$$

where A, X, B are as previously defined and L is a halogen atom. The napthols and activated aryl halides are commercially available or may be readily prepared by conventional methods from commercially available materials.

The compounds of formula 1 wherein n=0, Z=0, and $R_9$=H may be likewise synthesized from the isocyanate:

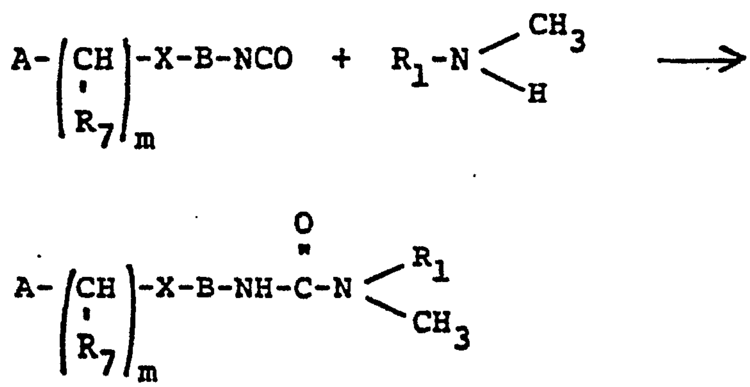

These isocyanates are prepared by conventional methods of reacting the corresponding aniline 8 with phosgene. The anilines 8 are obtained by reacting the appropriate naphthalkanols with activated arylhalides followed by reduction of the nitrophenyl ethers by chemical methods or catalytic hydrogenation.

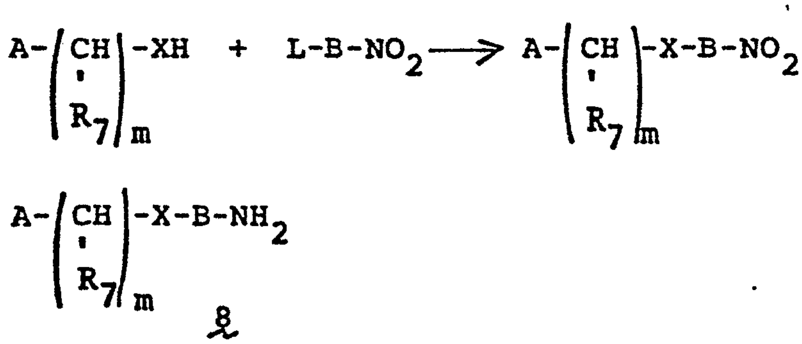

8

The required nitrophenyl ethers may also be prepared by reacting an appropriately activated naphthalkyl

derivative 9 with a nitrophenol in the presence of a strong base and in inert solvent:

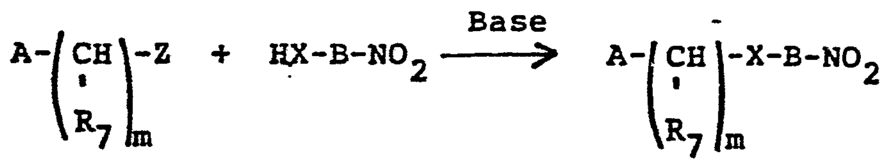


9 10

where Z may be a mesylate, tosylate, or chlorine, bromine, or iodine and A, R, X, and B are as previously defined.

The desired nitroethers 10 may also be prepared by reacting the alcohols 9 (Z=OH) with 4-halonitrobenzenes in the presence of a base, preferably with the addition of a phase transfer catalyst.

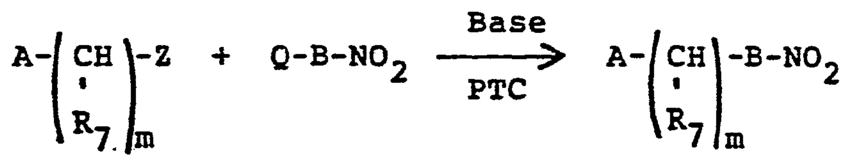


9 11

Where Q may be fluorine, bromine, chlorine, or iodine, and A, $R_7$, Z, B are as defined above. The base used in this reaction may be an alkali metal or alkaline earth metal hydroxide (e.g., sodium hydroxide, potassium hydroxide, calcium hydroxide, magnesium hydroxide), alkali metal or alkaline earth metal carbonates (e.g., sodium carbonate, potassium carbonate, calcium carbonate, magnesium carbonate), metal hydrides (e.g., sodium hydride, potassium hydride, lithium hydride), organic amines (e.g., pyridine, quinoline, 1,5-diazabicyclo [4.3.0] non-5-ene (DBN), metal alcoholates (potassium

tert-butoxide, potassium tert-amylate), alkali metal amides (e.g., sodium amide, lithium amide) and organolithium compounds (e.g., n-butyl lithium). In general, higher yields and fewer side products are obtained if a phase transfer catalyst (PTC) is used in the reaction. Examples of such catalysts include quaternary ammonium salts (e.g., tribenzylammonium bromide, tetraethylammonium chloride, tetrabutylammonium bromide) or crown ethers (e.g., dicyclohexyl-18-crown-6, dibenzo-18-crown-6).

The halo-4-nitrophenol 11 may be used as the free phenol or in the form of its salt. When it is used in the form of its lithium, potassium, or sodium salt, it is not necessary to use a base.

The solvent for the above reaction may be any of several organic polar solvents (e.g., tetrahydrofuran, dimethylsulfoxide, dimethylformamide) or non-polar organic solvents (e.g., benzene, toluene, petroleum ethers, diethyl ether, dimethoxyethane, etc.).

The reaction temperature depends upon the base, solvent, and substitution on the halonitrobenzene, but generally it is in the range of 0°C to 200°C.

The required anilines (7 or 8) are prepared by reduction of the nitrophenyl ethers. This reduction may be carried out with catalytic hydrogenation or with chemical reducing agents. In the former case, the catalyst may be palladium, nickel, copper, chromium, or platinum. The amount of catalyst is usually within the range of 0.01% to 10% by weight based on the substituted nitrobenzene. Solvents which may be used in the catalytic hydrogenation include methanol, ethanol,

acetic acid, toluene, diethyl ether and dioxane. The hydrogenation may be carried out at room temperature or with heating under atmospheric or higher pressures. Optimum conditions for the catalytic hydrogenation depend upon catalyst, solvent, and substrate.

The chemical reduction of the nitrophenyl ethers may be carried out by a variety of methods. The reducing agent may be any of several metals or their salts (e.g., iron, tin, zinc) and an appropriate acid (hydrochloric acid, acetic acid). Alternatively, a metal (iron, tin) and strong alkali may be used. In still another method, various sulfur compounds (sodium sulfide, sodium hydrogen sulfide, hydrogen sulfide, sodium dithionite, etc.) may be used. Finally, any number of well known reducing agents (e.g., sodium borohydride, lithium aluminum hydride, or sodium cyanoborohydride. ) may be used.

The phenyl isocyanates are obtained from the anilines by dissolving phosgene in an unreactive solvent (e.g. benzene, toluene, or ethyl acetate) and adding the aniline to this solvent at -20°C to 30°C. Reaction occurs to give the phenylisocyanate as the temperature is raised from 30°C to 110°C.

The following examples illustrate the best mode presently contemplated for the practice of this invention:

Example 123

N-[3-chloro-4-(5-chloro-8-quinyloxy)phenyl]-N'-methyl-N'-methoxy Urea

Part A: Synthesis of 4-(5-chloro-8-quinyloxy)-3-chloronitrobenzene.

A 2 liter round bottom flask equipped with a mechanical stirrer, thermometer, and reflux condenser with $N_2$ inlet was charged with 44.9 g (0.25 mol) of 5-chloro-8-hydroxyquinoline, 47.5 g (0.25 mol) of 3,4-dichloronitrobenzene, 48.4 g (0.35 mol) of anhydrous potassium carbonate, and 300 ml of dry DMF. The mixture was stirred and heated (oil bath) under nitrogen to 100°C for 12 hours. The mixture was cooled to room temperature and as much of the DMF as possible removed under reduced pressure. The residue was taken up in hot toluene ( 2 l) and filtered through a celite pad. The toluene was washed twice with water, twice with 5% NaOH, once with brine. The toluene was dried ($MgSO_4$) and removed under reduced pressure to leave 72.0 g of a crude solid. This was recrystallized from methylene chloride to give 66.3 g (94%) of a white crystalline solid, MP 170-171°C. The NMR was consistent with the desired product.

| Anal: | %C | %H | %N |
|---|---|---|---|
| Calcd. | 53.76 | 2.41 | 8.36 |
| Found | 53.50 | 2.40 | 8.42 |

Part B: Synthesis of 4-(5-chloro-8-quinyloxy)-2-chloroaniline.

A 1 liter round bottom flask was equipped with a mechanical stirrer, thermometer, and reflux condenser with $N_2$ inlet. The flask was charged with 79.9 g (0.422 mol) of tin (II) chloride; 71 ml of concentrated hydrochloric acid, and 60 mol of dioxane and the stirred mixture heated to 80°C. To this mixture was added in portions 30.0 g (0.105 mol) of 4-(5-chloro-8-quinyloxy)-3-chloronitrobenzene (from Part A). An exotherm was noted as the nitro compound was added.

The reaction mixture was heated to reflux for 30 min., cooled to room temperature and then cooled in an ice bath. A solution of 142 g of sodium hydroxide in 359 ml of water was added slowly to the stirred mixture. The mixture was filtered through Celite and then extracted three times with 400 ml of methylene chloride. This extract was dried ($MgSO_4$), treated with decolorizing charcoal, and the solvent removed under reduced pressure to leave 19.1 g (71%) of crude product as a green solid. This material was suitable for use in the next step without purification. NMR ($CDCl_3$, ): 5.08 (s broad, 2H); 6.61-9.32 (m,8H).

<u>Part C:</u> Synthesis of N-[3-chloro-4-(5-chloro-8-quinyloxy)]phenyl-N'-methyl-N-methoxy urea.

A 250 ml round bottom flask was equipped with a Dewar Dry-Ice condenser with drying tube, a magnetic stirrer, and an additional funnel. The flask was charged with 62.0 ml of 0.95 M phosgene (0.0588 mol) in toluene and warmed until phosgene is seen to be refluxing from the Dewar condenser. At this point 5.0 g (0.0196 mol) of 4-(5-chloro-8-quinyloxy)-2-chloroaniline (from Part B) in 25 ml of

toluene was added dropwise. When addition was complete, the reaction was refluxed one hour. The excess phosgene and toluene were removed under reduced pressure. The residual isocyanate was taken up in 100 ml of toluene and cooled in an ice-water bath while being stirred under $N_2$. A suspension of 7.65 g (0.0784 mol) of O,N-dimethylhydroxylamine hydrochloride in 25 ml of toluene was stirred for 10 min. with 4.40 g (0.0784 mol) of powdered KOH, the KCl removed by filtration, and the toluene solution of O,N-dimethylhydroxylamine dried ($MgSO_4$), filtered, and added dropwise to the isocyanate solution. The mixture was stirred overnight at room temperature, the toluene removed under reduced pressure, and the residue taken up in methylene chloride. The methylene chloride was washed 3X with 5% HCl, 2X with water, dried ($MgSO_4$) and stripped to leave 4.6 g of crude product as a brown solid. This was recrystallized from hexane-ethyl acetate to give 2.5 g (33%) of the desired urea, MP 164-165°C. TLC (3:1 chloroform-acetonitrile) shows one spot at Rf 0.58.

| Anal. | %C | %H | %N |
|---|---|---|---|
| Calcd. | 55.12 | 3.85 | 10.71 |
| Found | 54.35 | 3.69 | 10.37 |

Example 18

N,N-dimethyl-4-[2-(1-naphth)ethyloxyphenyl Urea

Part A: Synthesis of 2-(1-naphth)ethyl tosylate.

A 500 ml round bottom flask was equipped with a magnetic stirrer and addition funnel with

$N_2$ inlet. The flask was charged with 56.7 g (0.291 mol) of p-toluenesulfonyl chloride and 75 ml of dry pyridine and cooled in an ice-water bath. To this solution was added, dropwise, 50.7 g (0.291 mol) of 1-naphthaleneethanol as a solution in the minimum amount of pyridine. The mixture was stirred overnight in the ice bath, poured on to ice and extracted into methylene chloride. The methyene chloride was washed several times with ice cold HCl (1N), then with water until neutral. The solution was dried ($MgSO_4$) then the solvent removed under reduced pressure to leave a light orange oil which slowly solidified. This material was recrystallized from hexane-methylene chloride to give 35.6 g (34%) of the desired tosylate as a white, crystalline solid, MP 61-63°C. NMR ($CDCl_3$, ): 2.30 (s,3H); 3.37 (t,2H); 4.28 (t,2H); 6.75-7.96 (m,11H).

Part B: Synthesis of 4-[(1-naphth)-ethyloxy]-nitrobenzene.

A 500 ml round bottom flask was equipped with an air condenser with $N_2$ inlet, mechanical stirrer, and addition funnel. The 18.1 g (0.0919 mol) of sodium 4-nitrophenoxide was dried by azeotroping with toluene until no water was observed in the distillate. The toluene was removed under reduced pressure and the salt immediately taken up in 150 ml of dry DMF and charged to the 500 ml flask. The 30.0 g (0.0919 mol) of 2-(1-naphth)ethyl tosylate (prepared in Part A) was taken up in 100 ml of dry DMF and added at a rapid drop rate to the salt solution. When addition was complete, the mixture was stirred at 100°C for 20 hours.

The mixture was cooled and most of the DMF

removed under reduced pressure. The residue was diluted with water, the solid product removed by filtration, washed thoroughly with water, and dried at room temperature to give 24.0 g (89%) of the desired product, MP 100-102°C. NMR ($CDCl_3$,δ): 3.50 (t,2H); 4.28 (t,2H); 6.63-8.30 (m,11H).

<u>Part C</u>: Synthesis of 4-[2-(1-naphth)-ethoxy]aniline.

Using the identical procedure described in Part B of Example 1, and using 24.0 g (0.0818 M) of 4-[(1-naphth)ethyloxy]nitrobenzene, 62 g of tin (II) chloride, 55 ml of 6N HCl, 85 ml of dioxane, 105 g of NaOH, and 260 ml of water, a total of 21.5 g of product ( 100%) was obtained as an oil. This material was used without purification. NMR ($CDCl_3$, ): 3.40 (t,2H); 3.62 (s,2H); 4.2 (t,2H); 6.38-8.26 (m,11H).

<u>Part D</u>: Synthesis of 4-[2-(1-naphth)ethyl-oxy]phenyl-N,N-dimethyl urea.

Using exactly the procedure outlined in Part C of Example 1, and starting with 5.0 g (0.0228 mol) of 4-[2-(1-naphth)ethoxy]aniline, 6.8 g (0.0684 mol) of phosgene in 100 ml of toluene, and 2.9 g (0.0644 mol) of dimethylamine in 50 ml of toluene, a total of 5.0 g (66%) of the desired urea was obtained as a white powder after recrystallization from toluene, MP 132-133°C.

| Anal. | %C | %H | %N |
|---|---|---|---|
| Calcd. | 75.42 | 6.63 | 8.42 |
| Found | 75.20 | 6.60 | 8.30 |

<u>Example 20</u>

<u>4-[2-(1-naphth)ethoxy]-3-chlorophenyl-N,O-dimethyl Urea</u>

Part A: Synthesis of 4-[2-(1-naphth)-ethoxy]-3-chloronitrobenzene.

A 2 liter round bottom flask equipped with a mechanical stirrer, reflux condenser with nitrogen inlet, thermometer, and addition funnel was charged with 100.0 g (0.581 mol) of 1-naphthaleneethanol, 16.3 g (0.0581 mol) of tetrabutylammonium chloride, 481 ml of toluene, and 139 ml of 50% aqueous sodium hydroxide. As the mixture was stirred vigorously under nitrogen, a solution of 111.5 g (0.581 mol) of 3,4-dichloronitrobenzene in 100 ml of toluene was added dropwise. When addition was complete, the mixture was heated with an oil bath to 50°C for 5 hours.

The mixture was poured into water then extracted thoroughly with methylene chloride. The methylene chloride solution was washed three times with water, dried ($MgSO_4$), and the solvent removed under reduced pressure. The resulting yellow solid was stirred with 3 l of hot water for 3 hr. to remove the remaining tetrabutylammonium chloride. The nitro ether was collected by filtration, dissolved in methylene chloride, dried ($MgSO_4$), and the solvent removed under reduced pressure. The residue was recrystallized from hexane-ethyl acetate to give 141.6 g (74%) of the desired product as a yellow crystalline solid, MP 120-122°C. NMR ($CDCl_3$, ): 3.56 (t,2H); 4.27 (t,3H); 6.73 (d,1H); 7.10-8.25 (m,9H).

Part B: Synthesis of 4-[2-(1-naphth)-ethoxy]-3-chloroaniline

Using the same procedure described in Part B of Example 1 and starting with 166.0 g (0.507 mol)

of 4-[2-(1-naphth)ethoxy]-3-chloronitrobenzene (from Part A above), 298.1 g (1.57 mol) of tin (II) chloride, 250 ml of dioxane, 280 ml of concentrated hydrochloric acid, and 150 ml of ethanol, a total of 140.0 g (93%) of the desired aniline was obtained as an oil which solidified upon standing. This material was used in the next step without purification. NMR ($CDCl_3$, ): 3.18 (s broad,2H); 3.50 (t,2H); 4.20 (t,2H); 6.21-6.90 (m,3H); 7.15-8.30 (m,7H).

Part C: Synthesis of 4-[2-(1-naphth)ethoxy]-3-chlorophenyl-N,O-dimethyl Urea.

Following the same procedure outlined in Part C of Example 1 and starting with 70 g (0.235 mol) of 4-[2-(1-naphth)ethoxy]-3-chloroaniline (from Part B above), 44 g (0.444 mol) of phosgene in toluene, 50.0 g (0.513 mol) of O,N-dimethylhydroxylamine hydrochloride, and 28.7 g (0.51 mol) of powdered potassium hydroxide, a total of 82 g of urea as crude product was obtained. This material was chromatographed through silica gel eluting with hexane-ethyl acetate to give 62 g of a light red oil. This was recrystallized from hexane-ethyl acetate to give 51.7 g (57%) of the desired urea as a white solid, MP 89-91°C.

| Anal. | %C | %H | %N |
|---|---|---|---|
| Calcd. | 65.53 | 5.51 | 7.28 |
| Found | 65.61 | 5.53 | 7.22 |

Example 55

4-[2-(5,6,7,8-tetrahydronaphthoxy)]phenyl-N,O-dimethyl Urea

Part A: Synthesis of 2-(5,6,7,8-tetrahydro-

naphthoxy)-4-nitrobenzene.

A 1 liter round bottom flask was equipped with a mechanical stirrer, reflux condenser with nitrogen inlet, and thermometer. The flask was chargea with 25.0 g (0.169 mol) of 5,6,7,8-tetrahydro-2-naphthol, 32.6 g (0.236 mol) of anhydrous potassium carbonate, and 200 ml of DMF. To the stirred solution was added, dropwise, 23.4 g (0.166 mol) of 4-nitro-1-fluorobenzene. The stirred mixture was heated to 100° for six hours.

The inorganic salts were removed by filtration, and most of the DMF taken off under reduced pressure. The residue was taken up in methylene chloride, washed with 1N sodium hydroxide, then 1 NHCl, and finally with water. The methylene chloride was dried ($Na_2SO_4$), treated with decolorizing charcoal, and the solvent removed under reduced pressure to leave an oil. This oil was recrystallized from toluene-hexane to give 32.3 g (71%) of the desired product as slightly yellow crystals, MP 73-76°C. NMR ($CDCl_3$, ): 1.72 (m,4H); 2.71 (m,4H); 6.90 (m,5H); 8.10 (m,2H).

<u>Part B</u>: Synthesis of 4-[2-(5,6,7,8-tetrahydronaphthoxy)]aniline.

Using the same procedure described in Part B of Example 1 and starting with 16.0 g (0.0595 mol) of 2-(5,6,7,8-tetrahydronaphthoxy)-4-nitrobenzene (from Part A above), 45.2 g (0.238 mol) of tin (II) chloride, 60 ml of p-dioxane, 40 ml of concentrated HCl, and 79.3 g of NaOH, a total of 11.5 g (81%) of the desired aniline was obtained by recrystallizing the crude product from toluene-hexane.

Part C: Synethsis of 4-[2-(5,6,7,8-tetrahydronaphthoxy)]phenyl-O,N-dimethyl Urea.

Using the procedure outlined in Part C of Example 1, and starting with 5.8 g (0.024 mol) of 4-[2-(5,6,7,8-tetrahydronaphthoxy)]aniline (from Part B above), 5.8 g (0.059 mol) of phosgene in 88 ml of ethyl acetate, and 2.95 g (0.0302 mol) of O,N-dimethylhydroxylamine hydrochloride, a total of 5.0 g (64%) of the desired urea was obtained after recrystallizing from toluene-hexane, MP 109-110°C.

| Anal. | %C | %H | %N |
|---|---|---|---|
| Calcd. | 69.92 | 6.79 | 8.58 |
| Found | 69.95 | 6.92 | 8.42 |

Example 67

4-[2-(5,6,7,8-tetrahydro)-1-naphthethyloxy]-phenyl-N,O-dimethyl Urea

Part A: Synthesis of 2-[(5,6,7,8-tetrahydro)-1-naphthalene]ethanol.

A Paar pressure bottle was charged with 50.0 g (0.291 mol) of 1-naphthaleneethanol, 1.5 g of Adam's catalyst, and 150 ml of glacial acetic acid. Hydrogenation at 45 psi and room temperature was carried out until no hydrogen uptake was observed. The catalyst was removed by filtration through Celite, the acetic acid removed under reduced pressure, and the residue taken up in methylene chloride. The methylene chloride was washed three times with 1 NNaOH, then water, dried ($MgSO_4$), and the solvent removed to leave a yellow oil. GC analysis of this oil showed it to be 85% pure desired product and 12% of an impurity, probably 2-[(1,2,3,4-tetrahydro)-1-naphthalene]ethanol. This

material was used without purification in the next step. NMR ($CDCl_3$, ): 1.76 (m,4H); 2.35 (s,1H); 2.68 (m,6H), 3.69 (t,2H); 6.95 (m,3H).

<u>Part B</u>: Synthesis of 4-[2-(5,6.7,8-tetrahydro)-1-naphthethyloxy]nitrobenzene (25-TNW-19).

Using the procedure described in Part A of Example 3 and starting with 18.5 g (0.105 mol) of 2-[(5,6,7,8-tetrahydro)-1-naphthalene]ethanol (from Part A above), 14.8 g (0.105 mol) of 1-fluoro-4-nitrobenzene, 24 g of 50% NaOH, (0.010 mol) of tetrabutylammonium bromide, and 125 ml of toluene, a total of 16.0 g (60%) of the desired material, MP 70-75°C was obtained after recrystallization from hexane-ethyl acetate. NMR ($CDCl_3$, ): 1.80 (m,4H); 2.75 (m,4H); 3.07 (t,2H); 4.20 (t,2H); 6.80-7.30 (m,5H); 8.15 (d,2H).

<u>Part C</u>: Synthesis of 4-[2-(5,6,7,8-tetrahydro)-1-naphthethyloxy]-aniline.

A Paar pressure bottle was charged with 16.0 g (0.0539 mol) of 4-[2-(5,6,7,8-tetrahydro)-1-naphthethyloxy]nitrobenzene (from Part B above), 1.7 g of 5% platinum on carbon, and 150 ml of toluene. The mixture was hydrogenated under 47 psi pressure at room temperature until no further uptake of hydrogen was observed ( 2 1/2 hrs.). The mixture was filtered through Celite to remove the catalyst and the toluene removed under reduced pressure to leave a pale yellow solid. NMR ($CDCl_3$, ): 1.80 (m,4H); 2.70 (M,4H); 3.00 (t,2H); 3.23 (s broad,2H); 4.04 (t,2H); 6.43-7.32 (m,7H).

<u>Part D</u>: Synthesis of 4-[2-(5,6,7,8-tetrahydro)-1-naphthethyloxy]phenyl-N,O-dimethyl Urea.

Using the procedure described in Part C of Example 1 and starting with 7.2 g (0.0270 mol) of 4-[2-(5,6,7,8-tetrahydro-1-naphthyloxy]aniline (from Part C above) and 6.65 g (0.0670 mol) of phosgene in toluene, and 6.0 g (0.0616 mol) of N,O-dimethylhydroxylamine hydrochloride, a total of 6.52 g. (68.5%) of the desired urea was obtained after recrystallization from hexane-ethyl acetate. The material was a white, crystalline solid, MP 108-109°C.

| Anal. | %C | %H | %N |
|---|---|---|---|
| Calcd. | 71.16 | 7.39 | 7.90 |
| Found | 70.63 | 7.67 | 7.70 |

Examples 1-158

In a manner similar to that employed in the preceding examples, and using one of the synthesis schemes previously disclosed, other urea compounds were prepared. The identity of the substituents on the generic formulae and the analytical data are set forth in Table I-XXV below:

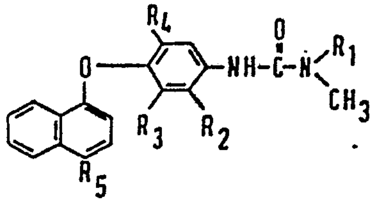

TABLE I

Physical Properties of Herbicidal Ureas

| Example | Molecular Formula | $R_5$ | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|
| 1 (1) | $C_{19}H_{18}N_2O_2$ | H | H | H | H | $CH_3$ | 148-150 | 74.49 | 5.92 | 9.14 | 72.07 | 6.11 | 8.82 |
| 2 | $C_{19}H_{16}Cl_2N_2O_2$ | H | H | Cl | Cl | $CH_3$ | 210-213 | 60.82 | 4.30 | 7.47 | 60.35 | 4.25 | 7.38 |
| 3 | $C_{19}H_{17}ClN_2O_2$ | H | H | H | Cl | $CH_3$ | 186-187.5 | 66.96 | 5.03 | 8.22 | 66.54 | 5.01 | 7.98 |
| 4 | $C_{19}H_{16}Cl_2N_2O_3$ | H | H | Cl | Cl | $OCH_3$ | 147-149 | 58.33 | 4.12 | 7.16 | 58.29 | 4.10 | 7.10 |
| 5 | $C_{19}H_{17}ClN_2O_3$ | H | H | H | Cl | $OCH_3$ | 123-125 | 63.96 | 4.80 | 7.85 | 63.80 | 4.84 | 7.63 |
| 6 | $C_{19}H_{16}Cl_2N_2O_3$ | Cl | H | H | Cl | $OCH_3$ | 143-145 | 58.33 | 4.12 | 7.16 | 58.15 | 4.12 | 7.01 |
| 7 | $C_{19}H_{16}Cl_2N_2O_2$ | Cl | H | H | Cl | $CH_3$ | 215-218 | 60.81 | 4.30 | 7.47 | 60.31 | 4.21 | 7.33 |
| 8 | $C_{19}H_{15}Cl_3N_2O_2$ | Cl | H | Cl | Cl | $CH_3$ | 248-252 | 55.70 | 3.69 | 6.84 | 55.18 | 3.62 | 6.80 |
| 9 | $C_{19}H_{15}Cl_2N_2O_3$ | Cl | H | Cl | Cl | $OCH_3$ | 151-153 | 53.61 | 3.55 | 6.58 | 53.18 | 3.52 | 6.39 |
| 10 | $C_{20}H_{16}ClF_3N_2O_2$ | Cl | H | H | $CF_3$ | $CH_3$ | 169-173 | 58.76 | 3.95 | 6.85 | 58.61 | 3.92 | 6.85 |
| 11 | $C_{20}H_{16}ClF_3N_2O_3$ | Cl | H | H | $CF_3$ | $OCH_3$ | 141-142 | 56.55 | 3.80 | 6.60 | 55.71 | 3.74 | 6.51 |
| 12 | $C_{20}H_{17}F_3N_2N_2$ | H | H | H | $CF_3$ | $CH_3$ | 130-132 | 64.17 | 4.58 | 7.48 | 63.98 | 4.64 | 7.46 |
| 13 | $C_{20}H_{17}F_3N_2O_3$ | H | H | H | $CF_3$ | $OCH_3$ | 104-106 | 61.54 | 4.39 | 7.18 | 61.35 | 4.21 | 6.84 |

(1) see U.S. Patent 4,280,835

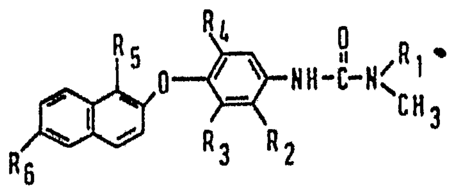

**TABLE II**

**Physical Properties of Herbicidal Ureas**

| Example | Molecular Formula | $R_6$ | $R_5$ | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|---|
| 14 | $C_{21}H_{21}ClN_2O_3$ | H | H | $CH_3$ | Cl | $CH_3$ | $CH_3$ | 199-201 | 68.38 | 5.74 | 7.60 | 68.40 | 5.87 | 7.33 |
| 15 | $C_{21}H_{21}ClN_2O_3$ | H | H | $CH_3$ | Cl | $CH_3$ | $OCH_3$ | 112-113 | 65.54 | 5.54 | 7.28 | 65.57 | 5.56 | 7.02 |
| 16 | $C_{19}H_{16}Br_2N_2O_2$ | Br | Br | H | H | H | $CH_3$ | 239-242 | 49.17 | 3.48 | 6.04 | 48.66 | 3.49 | 6.02 |
| 17 | $C_{19}H_{16}Br_2N_2O_3$ | Br | Br | H | H | H | $OCH_3$ | 152-154 | 47.53 | 3.36 | 5.83 | 48.75 | 3.46 | 5.95 |

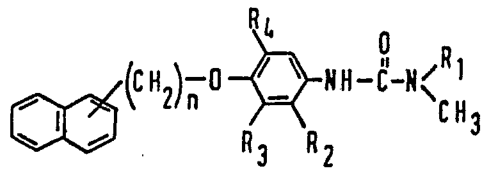

**TABLE III**

**Physical Properties of Herbicidal Ureas**

| Example | Molecular Formula | Ring (1) Position | n | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|---|
| 18 | $C_{21}H_{22}N_2O_3$ | 1 | 2 | H | H | H | $CH_3$ | 132-133 | 75.43 | 6.63 | 8.38 | 75.25 | 6.61 | 8.33 |
| 19 | $C_{21}H_{22}N_2O_3$ | 1 | 2 | H | H | H | $OCH_3$ | 85-86 | 71.98 | 6.33 | 8.00 | 71.83 | 6.40 | 7.83 |
| 20 | $C_{21}H_{21}ClN_2O_2$ | 1 | 2 | H | H | Cl | $CH_3$ | 142-143 | 68.37 | 5.75 | 7.60 | 67.88 | 5.95 | 7.08 |
| 21 | $C_{21}H_{21}ClN_2O_3$ | 1 | 2 | H | H | Cl | $OCH_3$ | 87-89 | 65.53 | 5.51 | 7.28 | 65.54 | 5.60 | 7.05 |
| 22 | $C_{21}H_{21}ClN_2O_3$ | 2 | 2 | H | H | Cl | $OCH_3$ | 116-117 | 65.53 | 5.51 | 7.28 | 66.26 | 5.81 | 6.96 |
| 23 | $C_{21}H_{21}ClN_2O_2$ | 2 | 2 | H | H | Cl | $CH_3$ | 132-134 | 68.37 | 5.75 | 7.60 | 68.37 | 5.84 | 7.39 |
| 24 | $C_{22}H_{21}F_3N_2O_2$ | 1 | 2 | H | H | $CF_3$ | $CH_3$ | 123-124 | 65.66 | 5.26 | 6.96 | 65.58 | 5.88 | 6.76 |
| 25 | $C_{22}H_{21}F_3N_2O_3$ | 1 | 2 | H | H | $CF_3$ | $OCH_3$ | 77-79 | 63.15 | 5.06 | 6.70 | 61.09 | 5.35 | 6.30 |
| 26 | $C_{21}H_{22}N_2O_2$ | 2 | 2 | H | H | H | $CH_3$ | 158-159 | 75.42 | 6.63 | 8.38 | 75.83 | 6.84 | 8.33 |
| 27 | $C_{21}H_{22}N_2O_3$ | 2 | 2 | H | H | H | $OCH_3$ | 98-100 | 71.98 | 6.33 | 7.99 | 70.84 | 6.17 | 7.57 |
| 28 | $C_{22}H_{21}F_3N_2O_2$ | 2 | 2 | H | H | $CF_3$ | $CH_3$ | 127-128 | 65.66 | 5.26 | 6.96 | 65.49 | 5.50 | 6.95 |
| 29 | $C_{22}H_{21}F_3N_2O_3$ | 2 | 2 | H | H | $CF_3$ | $OCH_3$ | 100-103 | 63.15 | 5.06 | 6.70 | 63.22 | 5.14 | 6.67 |
| 30 | $C_{20}H_{20}N_2O_2$ | 1 | 1 | H | H | H | $CH_3$ | 181-188 | 74.98 | 6.29 | 8.74 | 74.98 | 6.29 | 8.74 |
| 31 | $C_{20}H_{20}N_2O_3$ | 1 | 1 | H | H | H | $OCH_3$ | 143-144 | 71.41 | 5.99 | 8.33 | 70.49 | 5.97 | 7.81 |

(1) Attachment to naphthyl ring at position indicated.

**TABLE III (cont'd)**

**Physical Properties of Herbicidal Ureas**

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 39A | $C_{23}H_{26}N_2O_2$ | 1 | 4 | H | H | H | $CH_3$ | 88-92 | 76.21 | 7.23 | 7.73 | 75.93 | 7.33 | 7.88 |
| 39B | $C_{23}H_{26}N_2O_3$ | 1 | 4 | H | H | H | $OCH_3$ | oil | 72.99 | 6.93 | 7.40 | 72.40 | 6.97 | 7.48 |
| 39C | $C_{23}H_{25}ClN_2O_2$ | 1 | 4 | H | H | Cl | $CH_3$ | 105-109 | 69.60 | 6.35 | 7.06 | 69.72 | 6.40 | 6.760 |
| 39D | $C_{23}H_{25}ClN_2O_3$ | 1 | 4 | H | H | Cl | $OCH_3$ | 107-110 | 66.90 | 6.10 | 6.78 | 65.97 | 5.86 | 6.62 |
| 39E | $C_{24}H_{25}F_3N_2O_2$ | 1 | 4 | H | H | $CF_3$ | $CH_3$ | 108-112 | 66.96 | 5.85 | 6.51 | 63.90 | 5.63 | 6.90 |
| 39F | $C_{24}H_{25}F_3N_2O_3$ | 1 | 4 | H | H | $CF_3$ | $OCH_3$ | oil | 64.56 | 5.64 | 6.27 | 61.09 | 5.45 | 6.78 |
| 39G | $C_{22}H_{24}N_2O_3$ | 1 | 3 | H | H | H | $OCH_3$ | 95-98 | 72.51 | 6.64 | 7.69 | 73.05 | 6.81 | 7.52 |
| 39H | $C_{22}H_{23}ClN_2O_2$ | 1 | 3 | H | H | Cl | $CH_3$ | 98-101 | 69.01 | 6.05 | 7.32 | 68.37 | 6.23 | 7.04 |
| 39I | $C_{22}H_{23}ClN_2O_3$ | 1 | 3 | H | H | Cl | $OCH_3$ | 105-107 | 66.24 | 5.81 | 7.02 | 63.35 | 5.76 | 6.50 |
| 39J | $C_{23}H_{23}F_3N_2O_2$ | 1 | 3 | H | H | $CF_3$ | $CH_3$ | 95-96 | 66.34 | 5.57 | 6.73 | 65.20 | 5.54 | 6.51 |
| 39K | $C_{23}H_{23}F_3N_2O_3$ | 1 | 3 | H | H | $CF_3$ | $OCH_3$ | 79-80 | 63.88 | 5.36 | 6.48 | 64.11 | 5.50 | 6.49 |
| 39L | $C_{24}H_{27}ClN_2O_2$ | 1 | 5 | H | H | Cl | $CH_3$ | oil | 70.10 | 6.57 | 6.81 | 70.35 | 6.42 | 6.75 |
| 39M | $C_{24}H_{27}ClN_2O_3$ | 1 | 5 | H | H | Cl | $OCH_3$ | oil | - | - | - | - | - | - |
| 39N | $C_{20}H_{20}N_2O_2$ | 2 | 1 | H | H | H | $CH_3$ | 171-172 | 74.98 | 6.29 | 8.74 | 75.03 | 6.41 | 8.76 |
| 39O | $C_{20}H_{20}N_2O_3$ | 2 | 1 | H | H | H | $OCH_3$ | 119-121 | 71.41 | 5.99 | 8.33 | 71.74 | 6.23 | 8.34 |

[1] Attachment to naphthyl ring at position indicated.

**TABLE III** (cont'd)

**Physical Properties of Herbicidal Ureas**

| Example | Molecular Formula | Ring Position (1) | n | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|---|
| 32 | $C_{20}H_{19}ClN_2O_2$ | 1 | 1 | H | H | Cl | $CH_3$ | 181-182 | 67.69 | 5.39 | 7.89 | 67.63 | 5.68 | 7.88 |
| 33 | $C_{20}H_{19}ClN_2O_3$ | 1 | 1 | H | H | Cl | $OCH_3$ | 132-133 | 64.77 | 5.16 | 7.55 | 64.01 | 5.34 | 7.57 |
| 34 | $C_{21}H_{19}F_3N_2O_2$ | 1 | 1 | H | H | $CF_3$ | $CH_3$ | 200-200 | 64.94 | 4.93 | 7.21 | 64.47 | 5.01 | 6.99 |
| 35 | $C_{21}H_{19}F_3N_2O_3$ | 1 | 1 | H | H | $CF_3$ | $OCH_3$ | 154-155 | 62.35 | 4.73 | 6.93 | 61.99 | 4.90 | 6.89 |
| 36 | $C_{22}H_{24}N_2O_2$ | 1 | 2 | H | H | $CH_3$ | $CH_3$ | 145-147 | 75.83 | 6.94 | 8.04 | 75.90 | 7.01 | 7.93 |
| 37 | $C_{22}H_{24}N_2O_3$ | 1 | 2 | H | H | $CH_3$ | $OCH_3$ | oil | 72.50 | 6.63 | 7.68 | 70.07 | 6.53 | - |
| 38 | $C_{21}H_{21}FN_2O_3$ | 1 | 2 | H | H | F | $OCH_3$ | 104-105 | 68.46 | 5.74 | 7.60 | 68.71 | 5.92 | 7.52 |
| 39 | $C_{21}H_{21}FN_2O_2$ | 1 | 2 | H | H | F | $CH_3$ | 135-137 | 71.57 | 6.00 | 7.94 | 71.78 | 6.22 | 7.94 |

(1) Attachment to tetrahydronaphthyl ring at position indicated.

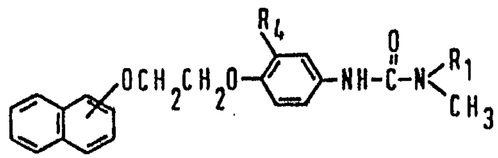

**TABLE IV**

**Physical Properties of Herbicidal Ureas**

| Example | Molecular Formula | Ring Position | $R_4$ | $R_1$ | M.P. (C.) | Calculated C | Calculated H | Calculated N | Found C | Found H | Found N |
|---|---|---|---|---|---|---|---|---|---|---|---|
| 40 | $C_{21}H_{22}N_2O_3$ | 1 | H | $CH_3$ | 111-114 | 71.98 | 6.33 | 8.00 | 70.15 | 6.36 | 7.72 |
| 41 | $C_{21}H_{22}N_2O_4$ | 1 | H | $OCH_3$ | 119-122 | 68.84 | 6.05 | 7.65 | 67.25 | 6.19 | 7.51 |
| 42 | $C_{21}H_{22}N_2O_3$ | 2 | H | $CH_3$ | 182-185 | 71.98 | 6.33 | 8.00 | 70.49 | 6.43 | 7.70 |
| 43 | $C_{21}H_{22}N_2O_4$ | 2 | H | $OCH_3$ | 147-150 | 68.84 | 6.05 | 7.65 | 67.40 | 6.01 | 7.64 |
| 44 | $C_{21}H_{21}ClN_2O_4$ | 2 | Cl | $OCH_3$ | 195-200 | 62.92 | 5.28 | 6.99 | 63.46 | 5.09 | 6.26 |
| 45 | $C_{21}H_{21}ClN_2O_3$ | 2 | Cl | $CH_3$ | 148-151 | 65.54 | 5.50 | 7.28 | 64.74 | 5.67 | 6.78 |
| 46 | $C_{21}H_{21}ClN_2O_3$ | 1 | Cl | $OCH_3$ | 118-121 | 62.92 | 5.28 | 6.99 | 62.71 | 5.11 | 6.95 |
| 47 | $C_{21}H_{21}ClN_2O_4$ | 1 | Cl | $CH_3$ | 124-127 | 65.54 | 5.50 | 7.28 | 64.62 | 5.45 | 6.73 |

(1) Attachment to naphthyl ring at position indicated.

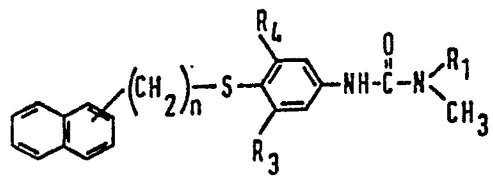

**TABLE V**

**Physical Properties of Herbicidal Ureas**

| Example | Molecular Formula | Ring(1) Position | n | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|
| 48 | $C_{21}H_{22}N_2OS$ | 1 | 2 | H | H | $CH_3$ | 156-157 | 71.96 | 6.33 | 7.99 | 71.79 | 6.47 | 7.95 |
| 49 | $C_{21}H_{22}N_2O_2S$ | 1 | 2 | H | H | $OCH_3$ | 104-105 | 68.82 | 6.05 | 7.64 | 68.93 | 6.25 | 7.60 |
| 50 | $C_{21}H_{21}ClN_2OS$ | 1 | 2 | H | Cl | $CH_3$ | 143-145 | 65.50 | 5.50 | 7.28 | 65.25 | 5.50 | 7.18 |
| 51 | $C_{21}H_{21}ClN_2O_2S$ | 1 | 2 | H | Cl | $OCH_3$ | 105-106 | 62.91 | 5.28 | 6.98 | 62.95 | 5.40 | 6.85 |

(1) Attachment to tetrahydronaphthyl ring at position indicated.

**TABLE V (cont'd)**

**Physical Properties of Herbicidal Ureas**

| Example | Molecular Formula | Ring Position[1] | n | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|
| 51A | $C_{22}H_{21}F_3N_2OS$ | 1 | 2 | H | $CF_3$ | $CH_3$ | 125-126 | 63.14 | 5.06 | 6.69 | 62.71 | 5.14 | 6.52 |
| 51B | $C_{22}H_{21}F_3N_2O_2S$ | 1 | 2 | H | $CF_3$ | $OCH_3$ | 99.5-100.5 | 60.82 | 4.87 | 6.45 | 60.48 | 4.85 | 6.34 |

[1] Attachment to naphthyl ring at position indicated.

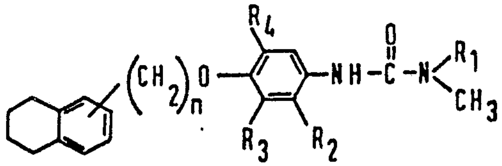

TABLE VI

Physical Properties of Herbicidal Ureas

| Example | Molecular Formula | Ring (1) Position | n | $R_2$ | $R_3$ | $R_4$ | $R_1$ | M.P. (°C.) | Calculated | | | Found | | |
|---|---|---|---|---|---|---|---|---|---|---|---|---|---|---|
| | | | | | | | | | C | H | N | C | H | N |
| 52 | $C_{19}H_{21}ClN_2O_2$ | 1 | 0 | H | H | Cl | $CH_3$ | 173-174 | 66.18 | 6.14 | 8.12 | 65.45 | 6.00 | 8.00 |
| 53 | $C_{19}H_{21}ClN_2O_3$ | 1 | 0 | H | H | Cl | $OCH_3$ | 84.0-86.5 | 63.25 | 5.87 | 7.76 | 61.11 | 6.02 | 7.25 |
| 54 | $C_{19}H_{22}N_2O_2$ | 2 | 0 | H | H | H | $CH_3$ | 180-182 | 73.52 | 7.14 | 9.03 | 73.56 | 7.16 | 8.95 |
| 55 | $C_{19}H_{22}N_2O_3$ | 2 | 0 | H | H | H | $OCH_3$ | 109-110 | 69.92 | 6.79 | 8.58 | 69.95 | 6.92 | 8.42 |
| 56 | $C_{20}H_{21}F_3N_2O_2$ | 2 | 0 | H | H | $CF_3$ | $CH_3$ | 173-174 | 63.48 | 5.59 | 7.40 | 63.64 | 5.64 | 7.29 |
| 57 | $C_{20}H_{21}F_3N_2O_3$ | 2 | 0 | H | H | $CF_3$ | $OCH_3$ | 126-127 | 60.91 | 5.37 | 7.10 | 61.09 | 5.37 | 6.93 |
| 58 | $C_{20}H_{21}F_3N_2O_3$ | 1 | 0 | H | H | $CF_3$ | $CH_3$ | 162-163 | 63.48 | 5.59 | 7.40 | 63.49 | 5.60 | 7.26 |
| 59 | $C_{20}H_{21}F_3N_2O_3$ | 1 | 0 | H | H | $CF_3$ | $OCH_3$ | 88-90 | 60.91 | 5.37 | 7.10 | 61.21 | 5.56 | 7.00 |
| 60 | $C_{19}H_{21}ClN_2O_2$ | 1 | 0 | H | H | Cl | $CH_3$ | 180-181 | 66.18 | 6.14 | 8.12 | 66.07 | 6.78 | 8.03 |
| 61 | $C_{19}H_{21}ClN_2O_3$ | 1 | 0 | H | H | Cl | $OCH_3$ | 107-108 | 63.24 | 5.87 | 7.76 | 63.43 | 6.01 | 7.57 |
| 62 | $C_{21}H_{25}ClN_2O_3$ | 2 | 0 | $CH_3$ | Cl | $CH_3$ | $OCH_3$ | 101-105 | 64.86 | 6.48 | 7.20 | 59.46 | 6.09 | 7.90 |
| 63 | $C_{19}H_{22}N_2O_2$ | 1 | 0 | H | H | H | $CH_3$ | 125-127 | 73.52 | 7.45 | 9.03 | 73.2 | 7.11 | 8.99 |
| 64 | $C_{19}H_{22}N_2O_3$ | 1 | 0 | H | H | H | $OCH_3$ | 120-122 | 69.92 | 6.80 | 8.58 | 70.57 | 7.08 | 8.26 |
| 65 | $C_{21}H_{25}ClN_2O_2$ | 2 | 0 | $CH_3$ | Cl | $CH_3$ | $CH_3$ | 174-178 | 67.64 | 6.76 | 7.51 | 65.49 | 6.70 | 7.81 |
| 66 | $C_{21}H_{26}N_2O_2$ | 1 | 2 | H | H | H | $CH_3$ | 127-128 | 74.52 | 7.74 | 8.28 | 74.20 | 7.50 | 8.02 |
| 67 | $C_{21}H_{26}N_2O_3$ | 1 | 2 | H | H | H | $OCH_3$ | 108-109 | 71.16 | 7.39 | 7.90 | 70.63 | 7.67 | 7.70 |

(1) attachment to tetrahydronaphthyl ring at position indicated.

**TABLE VI (cont'd)**

**Physical Properties of Herbicidal Ureas**

| Example | Molecular Formula | Ring Position (1) | n | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|---|
| 68 | $C_{21}H_{25}ClN_2O_2$ | 1 | 2 | H | H | Cl | $CH_3$ | 112-114 | 67.63 | 6.75 | 7.51 | 67.60 | 6.80 | 7.28 |
| 69 | $C_{21}H_{25}ClN_2O_3$ | 1 | 2 | H | H | Cl | $OCH_3$ | 74-75 | 64.85 | 6.48 | 7.20 | 64.95 | 6.44 | 7.16 |
| 70 | $C_{22}H_{25}F_3N_2O_2$ | 1 | 2 | H | H | $CF_3$ | $CH_3$ | 140-141 | 65.01 | 6.20 | 6.89 | 64.97 | 6.20 | 6.84 |
| 71 | $C_{22}H_{25}F_3N_2O_3$ | 1 | 2 | H | H | $CF_3$ | $OCH_3$ | 95-96 | 62.54 | 5.96 | 6.63 | 62.78 | 6.04 | 6.52 |
| 72 | $C_{20}H_{24}N_2O_3$ | 1 | 0 | H | H | $OCH_3$ | $CH_3$ | 172-175 | 70.56 | 7.10 | 8.22 | 67.76 | 7.18 | 7.21 |
| 73 | $C_{20}H_{24}N_2O_4$ | 1 | 0 | H | H | $OCH_3$ | $OCH_3$ | 133-135 | 67.39 | 6.78 | 7.86 | 65.56 | 6.90 | 7.11 |
| 74 | $C_{20}H_{24}N_2O_2$ | 1 | 0 | H | H | $CH_3$ | $CH_3$ | 173-176 | 74.04 | 7.45 | 8.63 | 73.82 | 7.51 | 8.41 |
| 75 | $C_{20}H_{24}N_2O_3$ | 1 | 0 | H | H | $CH_3$ | $OCH_3$ | 76-78 | 70.56 | 7.10 | 8.22 | 70.72 | 7.25 | 8.09 |
| 76 | $C_{20}H_{23}ClN_2O_2$ | 1 | 0 | $CH_3$ | H | Cl | $CH_3$ | 171-172 | 66.93 | 6.46 | 7.80 | 67.02 | 6.51 | 7.69 |
| 77 | $C_{20}H_{23}ClN_2O_3$ | 1 | 0 | $CH_3$ | H | Cl | $OCH_3$ | 112-113 | 64.07 | 6.18 | 7.47 | 64.19 | 6.30 | 7.28 |
| 78 | $C_{21}H_{26}N_2O_3$ | 1 | 0 | $CH_3$ | H | $CH_3$ | $OCH_3$ | 97-98 | 71.15 | 7.39 | 7.90 | 70.89 | 7.44 | 7.79 |
| 79 | $C_{20}H_{24}N_2O_2$ | 1 | 1 | H | H | H | $CH_3$ | 175-176 | 74.04 | 7.45 | 8.63 | 73.68 | 7.41 | 8.59 |
| 80 | $C_{20}H_{24}N_2O_3$ | 1 | 1 | H | H | H | $OCH_3$ | 91-92 | 70.56 | 7.10 | 8.22 | 70.56 | 7.10 | 8.20 |

(1) Attachment to tetrahydronaphthyl ring at position indicated.

TABLE VI (cont'd)

Physical Properties of Herbicidal Ureas

| Example | Molecular Formula | Ring(1) Position | n | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|---|
| 81 | $C_{19}H_{21}FN_2O_2$ | 1 | 0 | H | H | F | $CH_3$ | 159-162 | 69.49 | 6.44 | 8.53 | 69.30 | 6.48 | 8.52 |
| 82 | $C_{19}H_{21}FN_2O_3$ | 1 | 0 | H | H | F | $OCH_3$ | 113-115 | 66.26 | 6.14 | 8.13 | 66.46 | 6.32 | 8.02 |
| 83 | $C_{21}H_{23}F_3N_2O_2$ | 1 | 1 | H | H | $CF_3$ | $CH_3$ | 192-194 | 64.27 | 5.90 | 7.13 | 62.85 | 5.90 | 6.69 |
| 84 | $C_{21}H_{23}F_3N_2O_3$ | 1 | 1 | H | H | $CF_3$ | $OCH_3$ | 157-158 | 61.75 | 5.67 | 6.86 | 61.55 | 5.80 | 6.71 |
| 85 | $C_{20}H_{24}N_2O_2$ | 1 | 0 | $CH_3$ | H | H | $CH_3$ | 112-113 | 74.04 | 7.45 | 8.63 | 74.01 | 7.47 | 8.61 |
| 86 | $C_{20}H_{24}N_2O_3$ | 1 | 0 | $CH_3$ | H | H | $OCH_3$ | 86-88 | 70.56 | 7.10 | 8.22 | 70.77 | 6.40 | 8.20 |
| 87 | $C_{20}H_{23}ClN_2O_2$ | 1 | 1 | H | H | Cl | $CH_3$ | 146-148 | 66.93 | 6.46 | 7.80 | 60.92 | 6.13 | 7.06 |
| 88 | $C_{20}H_{23}ClN_2O_3$ | 1 | 1 | H | H | Cl | $OCH_3$ | 135-137 | 64.07 | 6.18 | 7.47 | 63.43 | 6.29 | 7.35 |
| 89 | $C_{20}H_{24}N_2O_3$ | 2 | 0 | $CH_3$ | H | H | $OCH_3$ | 80-83 | 70.56 | 7.10 | 8.22 | 70.77 | 6.40 | 8.20 |
| 90 | $C_{20}H_{24}N_2O_2$ | 2 | 0 | $CH_3$ | H | H | $CH_3$ | 134-135 | 74.04 | 7.45 | 8.63 | 74.13 | 7.41 | - |
| 91 | $C_{19}H_{21}FN_2O_2$ | 2 | 0 | H | H | F | $CH_3$ | 168-169 | 69.49 | 6.44 | 8.53 | 69.10 | 6.39 | - |
| 92 | $C_{19}H_{21}FN_2O_3$ | 2 | 0 | H | H | F | $OCH_3$ | 110-112 | 66.26 | 6.14 | 8.13 | 66.14 | 6.08 | - |
| 93 | $C_{20}H_{24}N_2O_2$ | 2 | 0 | H | H | $CH_3$ | $CH_3$ | 158-160 | 74.04 | 7.45 | 8.63 | 74.13 | 7.41 | - |
| 94 | $C_{20}H_{23}ClN_2O_2$ | 2 | 0 | $CH_3$ | H | Cl | $CH_3$ | 140-141 | 66.93 | 6.46 | 7.80 | 66.95 | 6.42 | - |

(1) Attachment to tetrahydronaphthyl ring at position indicated.

**TABLE VI** (cont'd)

**Physical Properties of Herbicidal Ureas**

| Example | Molecular Formula | Ring(1) Position | n | $R_2$ | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|---|---|
| 95 | $C_{21}H_{26}N_2O_3$ | 2 | 0 | $CH_3$ | H | $CH_3$ | $OCH_3$ | | 71.15 | 7.39 | 7.90 | 71.50 | 7.32 | - |
| 96 | $C_{21}H_{26}N_2O_2$ | 2 | 0 | $CH_3$ | H | $CH_3$ | $CH_3$ | 90-93 | 74.52 | 7.74 | 8.27 | 74.55 | 7.70 | - |
| 97 | $C_{19}H_{20}Cl_2N_2O_2$ | 1 | 0 | H | H | Cl | $CH_3$ | 215-219 | 60.16 | 5.31 | 7.38 | 60.31 | 5.33 | 7.30 |
| 98 | $C_{19}H_{20}Cl_2N_2O_3$ | 1 | 0 | H | H | Cl | $OCH_3$ | 119-120 | 57.73 | 5.10 | 7.08 | 58.21 | 5.23 | 7.12 |

(1) Attachment to tetrahydronaphthyl ring at position indicated.

**TABLE VI (cont'd)**

**Physical Properties of Herbicidal Ureas**

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 98A | $C_{20}H_{23}ClN_2O_3$ | 2 | 0 | $CH_3$ | H | Cl | $OCH_3$ | 90-91 | 64.09 | 6.14 | - | 64.55 | 6.33 | - |
| 98B | $C_{20}H_{24}N_2O_3$ | 2 | 0 | $CH_3$ | H | H | $OCH_3$ | 93-94 | 70.06 | 7.05 | - | 71.21 | 7.27 | 1 |
| 98C | $C_{20}H_{21}N_3O_3$ | 1 | 0 | H | H | CN | $OCH_3$ | 139-140 | 68.37 | 5.98 | - | 68.33 | 6.11 | - |
| 98D | $C_{20}H_{21}N_3O_2$ | 1 | 0 | H | H | CN | $CH_3$ | oil | 71.64 | 6.26 | - | 71.62 | 6.44 | - |
| 98E | $C_{19}H_{21}N_3O_5$ | 1 | 0 | H | H | $NO_2$ | $OCH_3$ | 101-104 | 61.44 | 5.70 | 11.31 | 61.16 | 5.85 | 11.15 |

[1] Attachment to tetrahydronaphthyl ring at position indicated.

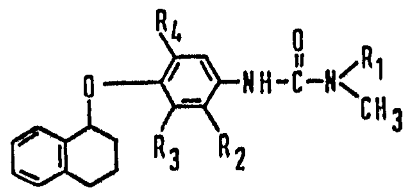

TABLE VII

Physical Properties of Herbicidal Ureas

| Example | Molecular Formula | $R_2$ | $R_3$ | $R_4$ | $R_1$ | M.P. (C.) | Calculated C | Calculated H | Calculated N | Found C | Found H | Found N |
|---|---|---|---|---|---|---|---|---|---|---|---|---|
| 99 | $C_{19}H_{22}N_2O_2$ | H | H | H | $CH_3$ | 145-146 | 73.51 | 7.14 | 9.02 | 73.44 | 7.33 | 9.06 |
| 100 | $C_{19}H_{22}N_2O_3$ | H | H | H | $OCH_3$ | 134-136 | 69.91 | 6.79 | 8.58 | 69.95 | 7.06 | 8.25 |
| 101 | $C_{19}H_{21}ClN_2O_3$ | H | H | Cl | $OCH_3$ | 134-136 | 63.24 | 5.86 | 7.76 | 62.56 | 5.85 | 8.05 |
| 102 | $C_{19}H_{21}ClN_2O_2$ | H | H | Cl | $CH_3$ | 152-154 | 66.17 | 6.13 | 8.12 | 65.33 | 6.15 | 7.97 |

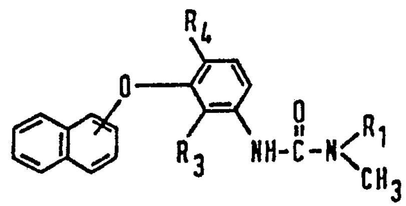

**TABLE VIII**

**Physical Properties of Herbicidal Ureas**

| Example | Molecular Formula | Ring(1) Position | $R_3$ | $R_4$ | $R_1$ | M.P. (°C.) | Calculated C | Calculated H | Calculated N | Found C | Found H | Found N |
|---|---|---|---|---|---|---|---|---|---|---|---|---|
| 103 | $C_{19}H_{22}N_2O_2$ | 1 | H | H | $CH_3$ | 139-141 | 73.51 | 7.14 | 9.02 | 73.51 | 7.32 | 9.22 |
| 104 | $C_{19}H_{22}N_2O_3$ | 1 | H | H | $OCH_3$ | 79-81 | 69.91 | 6.79 | 8.58 | 70.57 | 7.07 | 8.75 |

(1) Attachment to naphthyl ring at position indicated.

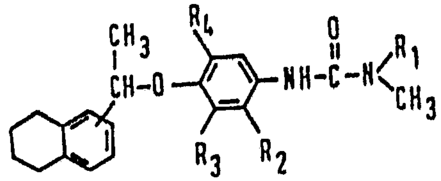

TABLE IX

Physical Properties of Herbicidal Ureas

| Example | Molecular Formula | Ring (1) Position | $R_2$ | $R_3$ | $R_4$ | $R_1$ | M.P. (C.) | Calculated C | Calculated H | Calculated N | Found C | Found H | Found N |
|---|---|---|---|---|---|---|---|---|---|---|---|---|---|
| 105 | $C_{21}H_{22}N_2O_2$ | 1 | H | H | H | $CH_3$ | 164-165 | 75.42 | 6.63 | 8.37 | 74.78 | 6.63 | 8.37 |
| 106 | $C_{21}H_{22}N_2O_3$ | 1 | H | H | H | $OCH_3$ | 147-148 | 71.97 | 6.32 | 7.99 | 71.97 | 6.44 | 7.62 |

(1) Attachment to tetrahydronaphthyl ring at position indicated.

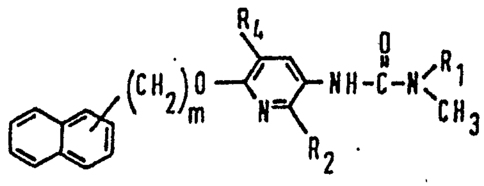

TABLE X

Physical Properties of Herbicidal Ureas

| Example | Molecular Formula | Ring(1) Position | n | $R_2$ | $R_4$ | $R_1$ | M.P. (C.) | Calculated C | Calculated H | Calculated N | Found C | Found H | Found N |
|---|---|---|---|---|---|---|---|---|---|---|---|---|---|
| 107 | $C_{20}H_{21}N_3O_2$ | 1 | 2 | H | H | $CH_3$ | 147-150 | 71.61 | 6.31 | 12.52 | 72.32 | 6.42 | 12.30 |
| 108 | $C_{21}H_{23}N_3O_2$ | 1 | 2 | H | $CH_3$ | $CH_3$ | 117-119 | 72.18 | 6.63 | 12.02 | 73.17 | 7.02 | 11.46 |
| 109 | $C_{20}H_{21}N_3O_3$ | 1 | 2 | H | H | $OCH_3$ | 103-104 | 68.35 | 6.02 | 11.95 | 68.57 | 6.06 | 11.99 |
| 110 | $C_{21}H_{23}N_3O_3$ | 1 | 2 | H | $CH_3$ | $OCH_3$ | 85-86 | 69.02 | 6.34 | 11.49 | 69.18 | 6.57 | 11.43 |
| 111 | $C_{20}H_{20}ClN_3O_3$ | 1 | 2 | H | Cl | $OCH_3$ | 98-100 | 62.25 | 5.22 | 10.89 | 62.32 | 5.32 | 10.85 |

(1) Attachment to tetrahydronaphthyl ring at position indicated.

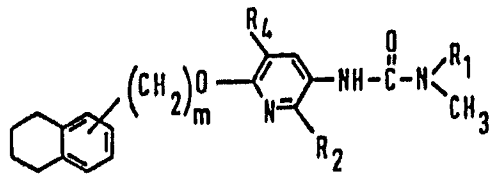

**TABLE XI**

**Physical Properties of Herbicidal Ureas**

| Example | Molecular Formula | Ring(1) Position | n | $R_2$ | $R_4$ | $R_1$ | M.P. (°C.) | Calculated C | Calculated H | Calculated N | Found C | Found H | Found N |
|---|---|---|---|---|---|---|---|---|---|---|---|---|---|
| 112 | $C_{18}H_{21}N_3O_2$ | 1 | 0 | H | H | $CH_3$ | 139-142 | 69.42 | 6.79 | 13.49 | 70.20 | 7.05 | 13.20 |
| 113 | $C_{18}H_{21}N_3O_3$ | 1 | 0 | H | H | $OCH_3$ | 87-89 | 66.03 | 6.46 | 12.83 | 65.62 | 6.57 | 12.60 |
| 114 | $C_{19}H_{19}N_3O_2$ | 1 | 2 | H | H | H | 154-156 | 71.00 | 5.95 | 13.07 | 70.84 | 6.10 | 13.22 |
| 115 | $C_{20}H_{20}ClN_3O_2$ | 1 | 2 | H | Cl | $CH_3$ | 147-149 | 64.94 | 5.45 | 11.36 | 65.60 | 5.73 | 11.47 |
| 116 | $C_{19}H_{23}N_3O_3$ | 1 | 0 | H | $CH_3$ | $OCH_3$ | 85-87 | 66.82 | 6.79 | 12.31 | 66.91 | 7.21 | 12.46 |
| 117 | $C_{19}H_{23}N_3O_2$ | 1 | 0 | H | $CH_3$ | $CH_3$ | 169-170 | 70.12 | 7.12 | 12.91 | 70.68 | 7.43 | 13.12 |
| 118 | $C_{18}H_{20}N_3O_2$ | 1 | 0 | H | Cl | $CH_3$ | 199-202 | 62.51 | 5.82 | 12.15 | 62.58 | 5.96 | 12.08 |
| 119 | $C_{18}H_{20}ClN_3O_3$ | 1 | 0 | H | Cl | $OCH_3$ | 109-112 | 59.74 | 5.57 | 11.61 | 60.00 | 5.80 | 11.64 |
| 120 | $C_{18}H_{21}N_3O_2$ | 2 | 0 | H | H | $CH_3$ | 163-172 | 69.42 | 6.79 | 13.49 | 69.73 | 7.09 | 13.78 |
| 121 | $C_{18}H_{21}N_3O_3$ | 2 | 0 | H | H | $OCH_3$ | 118-120 | 66.03 | 6.46 | 12.83 | 66.30 | 6.53 | 12.90 |

(1) Attachment to tetrahydronaphthyl ring at position indicated.

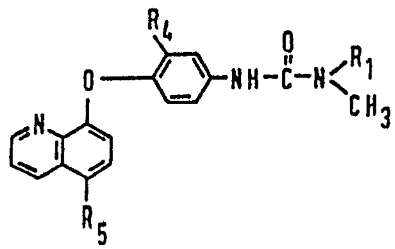

TABLE XII

Physical Properties of Herbicidal Ureas

| Example | Molecular Formula | $R_5$ | $R_4$ | $R_1$ | M.P. (C.) | Calculated C | Calculated H | Calculated N | Found C | Found H | Found N |
|---|---|---|---|---|---|---|---|---|---|---|---|
| 122 | $C_{18}H_{15}Cl_2N_3O_2$ | Cl | Cl | $CH_3$ | 77-80 | 57.47 | 4.02 | 11.17 | 56.77 | 4.01 | 10.46 |
| 123 | $C_{18}H_{15}Cl_2N_3O_3$ | Cl | Cl | $OCH_3$ | 164.165 | 55.12 | 3.85 | 10.71 | 54.35 | 3.69 | 10.37 |

The effectiveness of compounds representative of this invention as terrrestrial herbicides were evaluated as preemergence herbicides and postemergence herbicides. The test plants were mustard, nightshade, teaweed, velvetleaf and morninglory. For the preemergence test, seeds of the type of plants as shown in Tables XIII and XXV were sown in fresh soil. In the preemergence test, the soil was sprayed with a solution of the test compound immediately after the seeds were planted. The solution was about a 1% by weight solution of the test compound in acetone. The compounds were applied at the rate of 8 lbs/acre of soil surface, except where otherwise indicated in Tables XIII and XXV.

Approximately three weeks after spray applications, the herbicidal activity of the compound was determined by visual observation of the treated areas in comparison with untreated controls. These observations are reported in Tables XIII and XXV on a scale of 0 to 100% control of plant growth .

In the postemergence test the soil and developing plants were sprayed about two weeks after the seeds were sown. Except where indicated otherwise in Tables XIII and XXV the compounds were applied at the rate of 8 lbs/acre from about a 1% by weight solution of the test compound in acetone. The postemergence herbicidal activity was measured in the same way as the preemergence activity at three weeks following treatment.

The results of both the preemergence and postemergence tests are set forth in Tables XIII and XXV below:

**Table XIII**

**Biological Activity of Herbicidal Ureas**
**Percent Control**

| Example | Preemergent Herbicidal Activity | | | | | Postemergent Herbicidal Activity | | | | |
|---|---|---|---|---|---|---|---|---|---|---|
| | Morning | Velvet | Mustard | Nightshade | Teaweed | Morning | Velvet | Mustard | Nightshade | Teaweed |
| 1 | - | - | - | - | - | 30 | 100 | 100 | 100 | - |
| 2 | - | - | 15 | - | - | 10 | 20 | 85 | 25 | - |
| 3 | - | - | 100 | - | - | 100 | 100 | 100 | 100 | - |
| 4 | - | - | 25 | - | - | 10 | 30 | 100 | 100 | - |
| 5 | - | 10 | 15 | - | 10 | - | 100 | - | - | 100 |
| 6 | - | 10 | 30 | - | - | 40 | 100 | 100 | 100 | - |
| 7 | - | 10 | 30 | - | - | - | 10 | 70 | 35 | - |
| 8 | - | - | - | - | - | 10 | 20 | 85 | 90 | - |
| 9 | - | - | - | - | - | 10 | 90 | 100 | 100 | - |
| 10 | 10 | - | 35 | - | 10 | 20 | 85 | 100 | 100 | 99 |
| 11 | - | - | - | 10 | 40 | 25 | 85 | 100 | 100 | 95 |
| 12 | 15 | 10 | 90 | 15 | 10 | 25 | 100 | 100 | 100 | 99 |
| 13 | - | 10 | 25 | - | 10 | 15 | 100 | 100 | 100 | 99 |
| 14 | - | - | - | - | 10 | - | - | - | - | - |
| 15 | - | - | 10 | - | - | - | - | - | - | - |
| 16 | - | 30 | - | - | - | - | - | - | 10 | - |
| 17 | - | - | 10 | - | - | 40 | 75 | 100 | 90 | 100 |
| 18 | 15 | - | - | 10 | 10 | 100 | 100 | 100 | 100 | 100 |
| 19 | 20 | 20 | 100 | 45 | 15 | 100 | 100 | 100 | 100 | - |
| 20 | 20 | - | 100 | 55 | 10 | 100 | 100 | 100 | 25 | 80 |
| 21 | 15 | 10 | 100 | 75 | 10 | 100 | 100 | 100 | 100 | 90 |
| 22 | 15 | 10 | 10 | 50 | - | 100 | 100 | 100 | 100 | 100 |
| 23 | 10 | - | 10 | - | - | 15 | 25 | - | 100 | 100 |
| 24 | 10 | 10 | - | 10 | - | 100 | 99 | 100 | 100 | 100 |
| 25 | 40 | 10 | 60 | 45 | 15 | 100 | 99 | 100 | 100 | 100 |
| 26 | - | - | - | 70 | - | 10 | 80 | 100 | 90 | 60 |
| 27 | 65 | - | - | - | - | 100 | 100 | 100 | 65 | 100 |
| 28 | 55 | - | 100 | 100 | 60 | 100 | 100 | 100 | 100 | 100 |
| 29 | 20 | 10 | 80 | 10 | 10 | 100 | 85 | 100 | 100 | 100 |
| 30 | - | - | - | 10 | 10 | 80 | 95 | 100 | 100 | 20 |
| 31 | - | - | - | - | - | 100 | 100 | 100 | 100 | 70 |
| 32 | - | - | - | - | - | 35 | - | 100 | - | 25 |

A dash (-) indicates that compound not tested. A blank or zero means tested at concentration indicated but no response.

Table XIII(continued)

Biological Activity of Herbicidal Ureas
Percent Control

| Example | Preemergent Herbicidal Activity | | | | | Postemergent Herbicidal Activity | | | | |
|---|---|---|---|---|---|---|---|---|---|---|
| | Morning | Velvet | Mustard | Nightshade | Toeweed | Morning | Velvet | Mustard | Nightshade | Toeweed |
| 33 | 20 | - | - | - | - | 100 | - | 100 | - | 50 |
| 34 | - | - | - | - | - | 45 | 10 | 98 | 60 | 20 |
| 35 | - | - | 10 | - | - | 25 | 30 | 100 | 90 | 40 |
| 36 | - | - | - | 10 | - | 70 | 95 | 100 | 100 | 95 |
| 37 | 90 | - | 100 | 70 | 30 | 100 | 85 | 100 | 100 | 100 |
| 38 | 10 | - | 20 | - | - | 100 | 80 | 100 | 100 | 80 |
| 39 | 15 | 35 | 25 | - | 10 | 100 | 60 | 100 | 100 | 80 |
| 40 | 15 | - | - | 30 | 15 | 85 | 100 | 95 | 100 | 90 |
| 41 | - | - | - | - | - | 15 | 85 | 60 | 100 | 20 |
| 42 | - | - | - | - | - | - | 10 | - | 25 | 35 |
| 43 | - | - | - | - | - | 10 | - | 35 | 40 | 38 |
| 44 | - | - | - | - | 10 | 10 | 10 | 30 | - | - |
| 45 | 10 | - | - | - | - | 10 | 40 | 50 | 25 | 20 |
| 46 | 10 | - | - | - | - | 10 | 40 | 30 | 70 | 35 |
| 47 | 10 | - | - | 10 | - | 15 | 30 | 20 | 45 | 35 |
| 48 | 10 | 10 | 20 | 10 | 10 | 20 | 85 | 100 | 100 | 35 |
| 49 | - | - | 100 | - | - | 55 | 100 | 100 | 100 | 100 |
| 50 | - | - | - | 10 | - | 10 | 20 | 10 | 10 | 20 |
| 51 | - | - | 100 | - | 15 | 15 | 25 | - | 15 | 28 |
| 52 | - | - | 55 | - | - | 80 | 100 | 100 | 80 | - |
| 53 | - | - | 100 | 15 | 15 | - | 100 | 100 | - | - |
| 54 | | | 10 | 10 | - | 100 | 90 | 100 | 100 | 100 |
| 55 | 30 | 10 | 100 | 50 | 45 | 100 | 100 | 100 | 100 | 100 |
| 56 | 10 | - | 10 | - | - | 100 | 100 | 100 | 100 | 100 |
| 57 | - | - | - | 30 | 35 | 60 | 100 | 100 | 100 | 100 |
| 58 | 70 | - | 80 | 60 | 10 | 100 | 45 | 100 | 50 | 65 |
| 59 | 10 | 0 | 100 | - | - | 100 | 100 | 100 | 100 | 99 |
| 60 | - | - | 20 | 10 | 10 | 45 | 20 | 100 | 99 | 95 |
| 61 | 10 | - | 100 | 25 | 100 | 90 | 100 | 100 | 100 | 100 |
| 62 | - | 10 | - | 10 | - | - | - | - | - | - |
| 63 | 100 | 30 | 35 | 45 | 10 | - | 100 | 100 | 100 | 99 |
| 64 | - | 25 | - | 30 | - | 100 | 100 | 100 | 100 | 90 |

Table XIII (continued)

Biological Activity of Herbicidal Ureas
Percent Control

| Example | Preemergent Herbicidal Activity | | | | | Postemergent Herbicidal Activity | | | | |
|---|---|---|---|---|---|---|---|---|---|---|
| | Morning | Velvet | Mustard | Nightshade | Teaweed | Morning | Velvet | Mustard | Nightshade | Teaweed |
| 65 | -- | - | - | - | - | - | - | - | - | 55 |
| 66 | 30 | - | 100 | 15 | - | 100 | 100 | 100 | 100 | 100 |
| 67 | - | 10 | 10 | - | - | 100 | 98 | 100 | 100 | 45 |
| 68 | - | - | - | - | - | 100 | 99 | 100 | 100 | 100 |
| 69 | - | - | 100 | - | - | 100 | 98 | 100 | 100 | 100 |
| 70 | 15 | - | 100 | - | 10 | 70 | 20 | 100 | 100 | 100 |
| 71 | 10 | - | 50 | - | - | 99 | 65 | 100 | 100 | 100 |
| 72 | - | - | - | - | - | 40 | - | 100 | 100 | - |
| 73 | - | - | - | - | 15 | 40 | 15 | 100 | 100 | 100 |
| 74 | - | - | 100 | - | - | 45 | 15 | - | 100 | 80 |
| 75 | 10 | - | 100 | - | - | 100 | 100 | 100 | 100 | 100 |
| 76 | - | - | - | - | - | - | - | 100 | - | - |
| 77 | - | - | - | - | - | - | 15 | 40 | 15 | 25 |
| 78 | - | - | - | - | - | - | - | - | - | - |
| 79 | - | - | - | - | - | 15 | 15 | 100 | 100 | 15 |
| 80 | - | 10 | - | - | - | 85 | 95 | 100 | 100 | 95 |
| 81 | - | - | - | - | - | 15 | 50 | 100 | 100 | 100 |
| 82 | - | - | 100 | - | - | 99 | 85 | 100 | 100 | 99 |
| 83 | - | - | - | - | - | 10 | - | 100 | 100 | 60 |
| 84 | - | - | - | - | - | 15 | - | 100 | 60 | 30 |
| 85 | 10 | 10 | - | - | - | 10 | 10 | 10 | 10 | 15 |
| 86 | 10 | 10 | - | - | 15 | - | 20 | 10 | 50 | 25 |
| 87 | - | - | - | - | - | 45 | 15 | 100 | 90 | - |
| 88 | - | - | - | - | - | 65 | 45 | 100 | 100 | 80 |
| 89 | 10 | - | 10 | - | 30 | 30 | 50 | - | 90 | 35 |
| 90 | - | - | - | - | - | 20 | 45 | 20 | 60 | 30 |
| 91 | - | - | - | 10 | - | 100 | 85 | 100 | 100 | 99 |
| 92 | - | 10 | 20 | 45 | 70 | 100 | 100 | 100 | 100 | 100 |
| 93 | - | - | 20 | 15 | - | 100 | 80 | 100 | 100 | 100 |
| 94 | - | - | 50 | 100 | - | 20 | - | - | - | 10 |
| 95 | - | - | 99 | 10 | 10 | - | 10 | - | 10 | 10 |
| 96 | 10 | - | 15 | - | 10 | - | 10 | - | - | - |

Table XIII (continued)

Biological Activity of Herbicidal Ureas
Percent Control

| Example | Preemergent Herbicidal Activity: Morning | Velvet | Mustard | Nightshade | Teaweed | Postemergent Herbicidal Activity: Morning | Velvet | Mustard | Nightshade | Teaweed |
|---|---|---|---|---|---|---|---|---|---|---|
| 97 | - | - | - | - | - | - | - | - | 10 | 15 |
| 98 | - | - | 98 | - | 10 | 15 | - | 10 | 10 | 10 |
| 99 | 60 | - | 70 | 100 | - | 45 | 30 | 100 | 100 | 100 |
| 100 | - | - | - | - | - | - | 45 | 100 | 100 | |
| 101 | - | - | 30 | 10 | 10 | 100 | 35 | 100 | 100 | 85 |
| 102 | - | - | - | 10 | 10 | 100 | 80 | 100 | 100 | 95 |
| 103 | - | - | - | 10 | - | 20 | 45 | 100 | 100 | 15 |
| 104 | - | 25 | 10 | 10 | 10 | 60 | 70 | 100 | 100 | 60 |
| 105 | - | - | - | - | 15 | - | - | 100 | 100 | - |
| 106 | - | 10 | - | - | - | - | 25 | - | 15 | - |
| 107 | 50 | - | 70 | 10 | - | 25 | 100 | 100 | 100 | 85 |
| 108 | 30 | - | 70 | 10 | 10 | 20 | 98 | 100 | 100 | 100 |
| 109 | 30 | 15 | 10 | 10 | 10 | 85 | 100 | 100 | 100 | 100 |
| 110 | - | - | 25 | - | - | 100 | 100 | 100 | 100 | 100 |
| 111 | - | 15 | 80 | - | 10 | 100 | 99 | 100 | 100 | 100 |
| 112 | 100 | - | 99 | 100 | - | 85 | 95 | 100 | 100 | 100 |
| 113 | 100 | 20 | 100 | 99 | - | 100 | 100 | 100 | 100 | 100 |
| 114 | - | - | 10 | 10 | - | - | 35 | - | 10 | 45 |
| 115 | - | - | - | - | - | 25 | 45 | 100 | 100 | 25 |
| 116 | 10 | 10 | - | - | - | 35 | 35 | 100 | 100 | 99 |
| 117 | 10 | - | - | 15 | - | 10 | - | 100 | 100 | 10 |
| 118 | - | - | - | - | - | 15 | - | 100 | 100 | - |
| 119 | - | 10 | 25 | - | - | 100 | 80 | 100 | 95 | 45 |
| 120 | 100 | 20 | 100 | 100 | 75 | - | 25 | 100 | 100 | 45 |
| 121 | 30 | 25 | 100 | 85 | 45 | 75 | 100 | 100 | 100 | 100 |
| 122 | 15 | - | 95 | 20 | 15 | 50 | 15 | 100 | 100 | 100 |
| 123 | - | - | - | - | - | 98 | 10 | 100 | 100 | 100 |

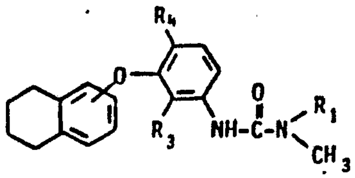

TABLE XIV

Physical Properties of Herbicidal Ureas

| Example | Molecular Formula | Ring Position[1] | $R_3$ | $R_4$ | $R_1$ | M.P. (°C) | Calculated C | Calculated H | Calculated N | Found C | Found H | Found N |
|---|---|---|---|---|---|---|---|---|---|---|---|---|
| 124 | $C_{19}H_{22}N_2O_3$ | 1 | H | H | $OCH_3$ | 79-81 | 69.96 | 6.74 | 8.58 | 70.57 | 7.07 | 8.75 |

[1] Attachment to tetrahydronaphthyl ring at position indicated.

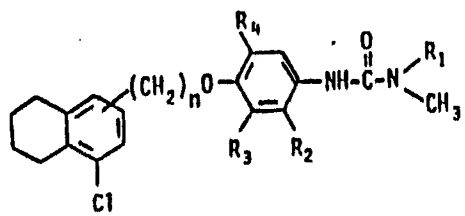

TABLE XV

Physical Properties of Herbicidal Ureas

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 125 | $C_{19}H_{20}Cl_2N_2O_2$ | 1 | 0 | H | H | Cl | $CH_3$ | 215-219 | 60.19 | 5.27 | 17.38 | 60.31 | 5.33 | 7.30 |
| 126 | $C_{19}H_{20}Cl_2N_2O_3$ | 3 | 0 | H | H | Cl | $OCH_3$ | 118.5-120 | 57.76 | 5.06 | 7.08 | 58.21 | 5.23 | 7.12 |

[1] Attachment to tetrahydronaphthyl ring at position indicated.

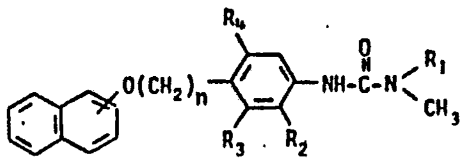

TABLE XVI

Physical Properties of Herbicidal Ureas

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 127 | $C_{21}H_{22}N_2O_3$ | 1 | 2 | H | H | H | $OCH_3$ | 81-84 | 71.98 | 6.33 | 8.00 | 71.58 | 6.51 | 8.07 |
| 128 | $C_{21}H_{22}N_2O_2$ | 1 | 2 | H | H | H | $CH_3$ | 155-157 | 75.46 | 6.58 | 8.37 | 76.28 | 6.89 | 8.24 |

[1] Attachment naphthyl ring at position indicated.

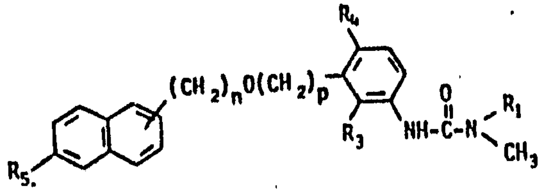

TABLE XVII

Physical Properties of Herbicidal Ureas

| Example | Molecular Formula | Ring Position[1] | n | p | $R_3$ | $R_4$ | $R_5$ | $R_1$ | M.P. (°C) | Calculated C | Calculated H | Calculated N | Found C | Found H | Found N |
|---|---|---|---|---|---|---|---|---|---|---|---|---|---|---|---|
| 129 | $C_{21}H_{22}N_2O_2$ | 1 | 1 | 1 | H | H | H | $CH_3$ | 129-131 | 75.46 | 6.58 | 8.37 | 75.40 | 6.80 | 8.34 |
| 130 | $C_{21}H_{22}N_2O_3$ | 1 | 1 | 1 | H | H | H | $OCH_3$ | 103-105 | 72.02 | 6.28 | 7.99 | 71.91 | 6.26 | 8.01 |
| 131 | $C_{22}H_{24}N_2O_2$ | 2 | 1 | 1 | H | H | $CH_3$ | $CH_3$ | 134-134.5 | 75.83 | 6.94 | 8.04 | 75.50 | 7.12 | 8.17 |
| 132 | $C_{22}H_{24}N_2O_3$ | 2 | 1 | 1 | H | H | $CH_3$ | $OCH_3$ | 100-101 | 72.51 | 6.64 | 7.69 | 71.17 | 6.67 | 7.63 |
| 133 | $C_{21}H_{21}ClN_2O_2$ | 2 | 1 | 0 | H | Cl | $CH_3$ | $CH_3$ | 176-177 | 68.38 | 5.74 | 7.59 | 67.81 | 5.82 | 7.46 |
| 134 | $C_{21}H_{21}ClN_2O_3$ | 2 | 1 | 0 | H | Cl | $CH_3$ | $OCH_3$ | 138-139 | 65.54 | 5.50 | 7.28 | 65.04 | 5.43 | 6.70 |
| 135 | $C_{21}H_{22}N_2O_2$ | 2 | 1 | 1 | H | H | H | $CH_3$ | 140-141 | 75.42 | 6.63 | 8.38 | 75.16 | 6.72 | 8.05 |
| 136 | $C_{21}H_{22}N_2O_3$ | 2 | 1 | 1 | H | H | H | $OCH_3$ | 76-77 | 71.98 | 6.33 | 7.99 | 71.90 | 6.45 | 7.86 |

[1] Attachment to naphthyl ring at position indicated.

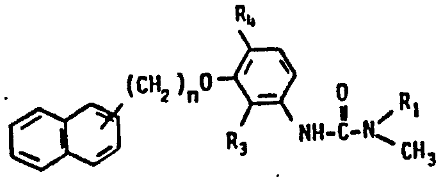

**TABLE XVIII**

**Physical Properties of Herbicidal Ureas**

| Example | Molecular Formula | Ring Position[1] | n | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|
| 137 | $C_{21}H_{22}N_2O_2$ | 1 | 2 | H | H | $CH_3$ | 110.5-113 | 75.42 | 6.63 | 8.38 | 75.93 | 6.89 | 8.43 |
| 138 | $C_{21}H_{22}N_2O_3$ | 1 | 2 | H | H | OCH | 86-89 | 71.98 | 6.33 | 7.99 | 72.12 | 6.43 | 7.81 |

[1] Attachment to naphthyl ring at position indicated.

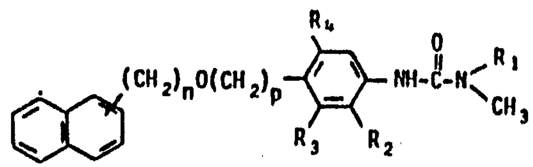

TABLE XIX

Physical Properties of Herbicidal Ureas

| Example | Molecular Formula | Ring Position[1] | n | p | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|---|
| 139 | $C_{21}H_{22}N_2O_2$ | 1 | 1 | 1 | H | H | H | $CH_3$ | 129-131 | 75.46 | 6.58 | 8.37 | 75.48 | 6.77 | 8.28 |
| 140 | $C_{21}H_{22}N_2O_3$ | 1 | 1 | 1 | H | H | H | $OCH_3$ | 71-72.5 | 72.02 | 6.28 | 7.99 | 71.94 | 6.53 | 7.94 |
| 141 | $C_{22}H_{24}N_2O_2$ | 1 | 2 | 1 | H | H | H | $CH_3$ | 108-110 | 75.86 | 6.90 | 8.05 | 74.02 | 7.42 | 8.07 |
| 142 | $C_{22}H_{24}N_2O_3$ | 1 | 2 | 1 | H | H | H | $OCH_3$ | oil | 72.53 | 6.60 | 7.30 | 72.24 | 6.85 | 7.90 |

1 Attachment to naphthyl ring at position indicated.

**TABLE. XX**

**Physical Properties of Herbicidal Ureas**

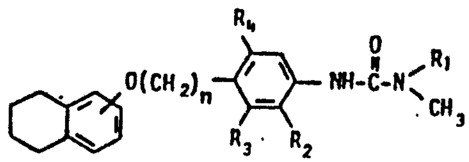

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 143 | $C_{20}H_{24}N_2O_3$ | 1 | 1 | H | H | H | $OCH_3$ | 122-124 | 70.57 | 7.11 | 8.23 | 70.83 | 7.23 | 7.97 |
| 144 | $C_{20}H_{24}N_2O_2$ | 1 | 1 | H | H | H | $CH_3$ | 130.5-133 | 74.05 | 7.46 | 8.64 | 73.86 | 7.70 | 8.43 |

[1] Attachment to naphthyl ring at position indicated.

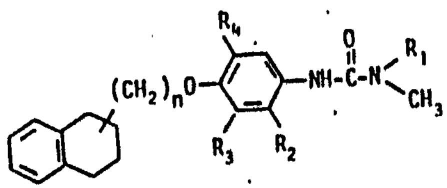

TABLE XXI

Physical Properties of Herbicidal Ureas

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 145 | $C_{21}H_{25}ClN_2O_3$ | 1 | 2 | H | H | Cl | $OCH_3$ | oil | 65.03 | 6.24 | 7.22 | 64.93 | 6.48 | 7.25 |

[1] Attachment to tetrahydronaphthyl ring at position indicated.

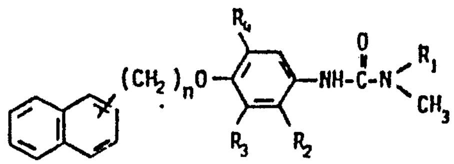

TABLE XXII

Physical Properties of Herbicidal Areas

| Example | Molecular Formula | Ring Position[1] | n | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|---|---|
| 146 | $C_{21}H_{24}ClN_2O_3$ | 1 | 2 | H | H | Cl | $OCH_3$ | oil | 65.20 | 5.99 | 7.24 | 63.21 | 5.75 | 6.93 |
| 147 | $C_{21}H_{24}N_2O_2$ | 1 | 2 | H | H | H | $CH_3$ | 124-125 | 74.97 | 7.19 | 8.33 | 74.60 | 7.17 | 8.24 |
| 148 | $C_{21}H_{24}N_2O_3$ | 1 | 2 | H | H | H | $OCH_3$ | 78-80 | 71.57 | 6.86 | 7.95 | 71.16 | 7.11 | 7.71 |

[1] Attachment to naphthyl ring at position indicated.

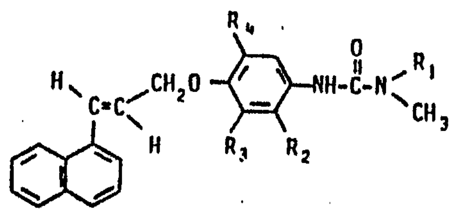

**TABLE XXIII**

**Physical Properties of Herbicidal Areas**

| Example | Molecular Formula | $R_2$ | $R_3$ | $R_4$ | $R_1$ | M.P. (°C) | Calculated C | Calculated H | Calculated N | Found C | Found H | Found N |
|---|---|---|---|---|---|---|---|---|---|---|---|---|
| 149 | $C_{22}H_{22}N_2O_3$ | H | H | H | $OCH_3$ | 92-95 | 72.91 | 6.12 | 7.73 | 71.91 | 5.99 | 7.53 |
| 150 | $C_{22}H_{21}ClN_2O_3$ | H | H | Cl | $OCH_3$ | 122-123 | 66.58 | 5.33 | 7.06 | 66.38 | 5.61 | 7.18 |

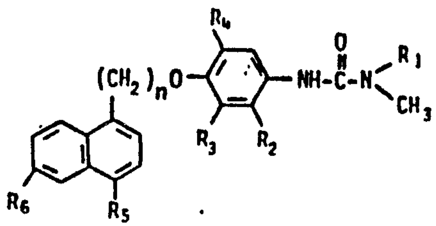

**TABLE XXIV**

**Physical Properties of Herbicidal Areas**

| Example | Molecular Formula | n | $R_2$ | $R_3$ | $R_4$ | $R_5$ | $R_6$ | $R_1$ | M.P. (°C) | Calculated C | Calculated H | Calculated N | Found C | Found H | Found N |
|---|---|---|---|---|---|---|---|---|---|---|---|---|---|---|---|
| 151 | $C_{22}H_{24}N_2O_2$ | 2 | H | H | H | $CH_3$ | H | $CH_3$ | 157-159 | 75.83 | 6.94 | 8.04 | 74.33 | 7.15 | 8.23 |
| 152 | $C_{22}H_{24}N_2O_3$ | 2 | H | H | H | $CH_3$ | H | $OCH_3$ | 101-102 | 72.51 | 6.64 | 7.69 | 72.57 | 6.58 | 7.83 |
| 153 | $C_{22}H_{23}ClN_2O_2$ | 2 | H | H | Cl | $CH_3$ | H | $CH_3$ | 127-128 | 69.01 | 6.05 | 7.32 | 68.83 | 6.09 | 7.35 |
| 154 | $C_{22}H_{23}ClN_2O_3$ | 2 | H | H | Cl | $CH_3$ | H | $OCH_3$ | 96-98 | 66.24 | 5.81 | 7.02 | 66.30 | 5.86 | 6.95 |
| 155 | $C_{22}H_{24}N_2O_2$ | 2 | H | H | H | H | $CH_3$ | $CH_3$ | 178-179 | 75.83 | 6.94 | 8.04 | 75.94 | 6.98 | 7.95 |
| 156 | $C_{22}H_{24}N_2O_3$ | 2 | H | H | H | H | $CH_3$ | $OCH_3$ | 125-127 | 72.51 | 6.64 | 7.69 | 72.63 | 6.60 | 7.37 |
| 157 | $C_{22}H_{23}ClN_2O_2$ | 2 | H | H | Cl | H | $CH_3$ | $CH_3$ | 144-145 | 69.01 | 6.05 | 7.32 | 68.85 | 6.19 | 7.18 |
| 158 | $C_{22}H_{23}ClN_2O_2$ | 2 | H | H | Cl | H | $CH_3$ | $OCH_3$ | 121-122 | 66.24 | 5.81 | 7.02 | 66.20 | 5.89 | 6.95 |

TABLE XXV

Biological Activity of Herbicidal Areas

Percent Control

| Example | Pre-emergent Herbicidal Control Morning | Velvet | Mustard | Nightshade | Teaweed | Post-emergent Herbicidal Control Morning | Velvet | Mustard | Nightshade | Teaweed |
|---|---|---|---|---|---|---|---|---|---|---|
| 39A | 100 | 100 | 100 | 100 | 100 | 100 | 100 | ---- | 100 | 100 |
| 39B | 50 | 62 | 100 | 100 | 54 | 100 | 97 | 100 | 100 | 100 |
| 39C | 100 | 100 | 100 | 100 | 99 | 100 | 100 | 100 | 100 | 100 |
| 39D | 0 | 45 | 100 | 99 | 6 | 26 | 100 | 100 | 100 | 100 |
| 39E | 100 | 100 | 100 | 100 | 100 | 75 | 100 | 100 | 100 | 93 |
| 39F | 6 | 100 | 100 | 100 | 98 | 20 | 100 | 100 | 100 | 100 |
| 39G | 0 | 0 | 0 | 0 | 0 | 86 | 100 | 100 | 100 | 86 |
| 39H | 0 | 0 | 0 | 33 | 45 | 14 | 100 | 100 | 100 | 100 |
| 39I | 0 | 0 | 0 | 0 | 6 | 38 | 76 | 100 | 100 | 100 |
| 39J | 0 | 0 | 100 | 35 | 10 | 38 | 22 | 100 | 100 | 10 |
| 39K | 0 | 0 | 20 | 0 | 0 | 40 | 54 | 100 | 100 | 35 |
| 39L | ---- | ---- | ---- | ---- | ---- | 0 | 0 | 10 | ---- | 60 |

TABLE XXV

**Biological Activity of Herbicidal Areas**
**Percent Control**

| Example | Pre-emergent Herbicidal Control | | | | | Post-emergent Herbicidal Control | | | | |
|---|---|---|---|---|---|---|---|---|---|---|
| | Morning | Velvet | Mustard | Nightshade | Teaweed | Morning | Velvet | Mustard | Nightshade | Teaweed |
| 39M | ---- | ---- | ---- | ---- | ---- | 10 | 20 | 96 | ---- | 81 |
| 39N | 26 | 0 | 19 | ---- | 6 | 88 | 100 | 100 | ---- | 100 |
| 39O | 12 | 6 | 0 | ---- | 0 | 80 | 100 | 100 | ---- | 62 |
| 51A | 0 | 0 | 0 | 0 | 0 | 0 | 50 | 100 | 100 | 43 |
| 51B | 0 | 0 | 0 | 0 | 0 | 0 | 55 | 100 | 100 | 32 |
| 98A | 0 | 0 | 100 | 6 | 0 | 0 | 56 | 100 | 26 | 0 |
| 98B | 0 | 76 | 100 | 12 | 56 | 100 | 100 | 100 | 100 | 100 |
| 98C | 0 | 0 | 0 | 0 | 0 | 0 | 0 | 0 | 0 | 0 |
| 98D | 0 | 0 | 0 | 0 | 0 | 0 | 44 | 20 | 40 | 100 |
| 98E | ---- | ---- | ---- | ---- | ---- | 0 | 11 | 12 | ---- | 70 |
| 124 | 0 | 76 | 100 | 12 | 56 | 100 | 100 | 100 | 100 | 100 |
| 125 | 0 | 0 | 0 | 0 | 0 | 0 | 0 | 0 | 0 | 10 |
| 126 | 0 | 0 | 99 | 0 | 6 | 6 | 0 | 0 | 0 | 0 |
| 127 | 55 | 0 | 100 | 0 | 0 | 0 | 87 | 100 | 100 | 78 |
| 128 | 0 | 0 | 0 | 0 | 0 | 10 | 0 | 100 | 100 | 0 |
| 129 | 6 | 0 | 100 | 20 | 0 | 72 | 100 | 100 | 100 | 87 |

TABLE XXV

**Biological Activity of Herbicidal Areas**
**Percent Control**

| Example | Pre-emergent Herbicidal Control | | | | | Post-emergent Herbicidal Control | | | | |
|---|---|---|---|---|---|---|---|---|---|---|
| | Morning | Velvet | Mustard | Nightshade | Teaweed | Morning | Velvet | Mustard | Nightshade | Teaweed |
| 130 | 0 | 0 | 100 | 0 | 0 | 100 | 100 | 100 | 100 | 100 |
| 131 | 0 | 0 | 0 | 0 | 0 | 44 | 32 | 100 | ----- | 74 |
| 132 | 0 | 0 | 10 | ----- | 12 | 32 | 98 | 100 | 100 | 100 |
| 133 | 0 | 0 | 0 | 0 | 0 | 100 | 40 | 100 | ----- | 6 |
| 134 | 0 | 0 | 0 | 0 | 0 | 100 | 100 | 100 | 0 | 84 |
| 135 | 12 | 0 | 90 | 0 | 0 | 100 | 100 | 100 | ----- | 100 |
| 136 | 0 | 0 | 100 | ----- | 0 | 100 | 100 | 100 | ----- | 100 |
| 137 | 42 | 30 | 100 | 75 | 0 | 30 | 100 | 100 | 100 | 100 |
| 138 | 12 | 19 | 100 | 12 | 87 | 76 | 100 | 100 | 100 | 100 |
| 139 | 100 | 0 | 100 | 6 | 12 | 96 | 100 | 100 | 100 | 84 |
| 140 | 58 | 100 | 100 | 100 | 10 | 98 | 85 | 100 | 100 | 10 |
| 141 | 0 | 0 | 0 | 0 | 0 | 100 | 100 | 100 | ----- | 10 |
| 142 | 0 | 0 | 100 | 30 | 0 | ----- | 100 | 100 | 100 | 100 |
| 143 | 0 | 0 | 0 | 0 | 0 | 12 | 14 | 62 | 38 | 33 |

TABLE XXV

Biological Activity of Herbicidal Areas
Percent Control

| Example | Pre-emergent Herbicidal Control | | | | | Post-emergent Herbicidal Control | | | | |
|---|---|---|---|---|---|---|---|---|---|---|
| | Morning | Velvet | Mustard | Nightshade | Teaweed | Morning | Velvet | Mustard | Nightshade | Teaweed |
| 144 | 20 | 100 | 54 | 12 | 0 | 100 | 100 | 100 | 100 | 26 |
| 145 | 0 | 0 | 100 | 100 | 6 | ---- | 100 | 100 | 100 | 100 |
| 146 | 0 | 0 | 54 | 0 | 0 | 40 | 100 | 100 | 100 | 100 |
| 147 | 100 | 32 | 100 | 100 | 12 | 100 | 100 | 100 | 100 | 100 |
| 148 | 0 | 100 | 0 | 100 | 78 | 100 | 100 | 100 | 100 | 100 |
| 149 | ---- | ---- | ---- | ---- | ---- | 6 | 0 | ---- | ---- | 12 |
| 150 | 0 | 0 | 0 | 0 | 0 | 0 | 53 | 100 | 100 | 84 |
| 151 | 78 | 86 | 100 | 0 | 30 | 100 | 100 | 100 | 100 | 100 |
| 152 | 19 | 0 | 100 | 0 | 12 | 100 | 100 | 100 | 55 | 100 |
| 153 | 0 | 6 | 0 | 0 | 0 | 65 | 54 | 100 | 100 | 100 |
| 154 | 42 | 12 | 56 | 100 | 12 | 43 | 75 | 100 | ---- | 100 |
| 155 | 0 | 0 | 0 | 0 | 0 | 26 | 0 | 76 | 0 | 0 |
| 156 | 0 | 0 | 6 | 0 | 0 | 0 | 30 | 100 | ---- | 30 |
| 157 | 0 | 0 | 74 | 0 | 0 | 62 | ---- | 100 | 100 | 53 |
| 158 | 0 | 0 | 0 | 0 | 0 | 20 | ---- | 100 | 100 | 65 |

It will be understood that the plant species employed in the above tests are merely representative of a wide variety of plants that can be controlled by the use of the compounds of this invention. The compounds contemplated in this invention may be applied as postemergent and preemergent herbicides according to methods known to those skilled in the art. Compositions containing the compounds as the active ingredient will usually comprise a carrier and/or diluent, either liquid or solid.

Suitable liquid diluents or carriers include water, petroleum distillates, or other liquid carriers with or without surface active agents. Liquid concentrates may be prepared by dissolving one of these compounds with a nonphytotoxic solvent such as acetone, xylene, or nitrobenzene and dispersing the toxicants in water with the aid of suitable surface active emulsifying and dispersing agent.

The choice of dispersing and emulsifying agents and the amount employed is dictated by the nature of the composition and the ability of the agent to facilitate the dispersion of the compound. Generally, it is desirable to use as little of the agent as is possible, consistent with the desired dispersion of the compound in the spray so that rain does not re-emulsify the compound after it is applied to the plant and wash it off the plant. Nonionic, anionic, amphoteric or cationic dispersing and emulsifying agents may be employed; for example, the condensation products of alkylene oxides with phenol and organic acids, alkyl aryl sulfonates, complex ether alcohols, or quaternary ammonium compounds.

In the preparation of wettable powder or dust or granulated compositions, the active ingredient is dispersed in and on an appropriately divided solid carrier such as clay, talc, bentonite, diatomaceous earth, or fullers earth. In the formulation of the wettable powders the aforementioned dispersing agents as well as lignosulfonates can be included.

The required amount of the compound contemplated herein may be applied per acre treated in from 1 to 200 gallons or more of liquid carrier and/or diluent or in from about 5 to 500 pounds of inert solid carrier and/or diluent. The concentration in the liquid concentrate will usually vary from about 10 to 95 percent by weight and in the solid formulations from about 0.5 to about 90 percent by weight. Satisfactory sprays, dusts, or granules for general use contain from about 1/4 to 15 pounds of active ingredient per acre.

The herbicides contemplated herein have a high margin of safety in that when used in sufficient amount to control broadleaf weeds they do not burn or injure the crop and they resist weathering which includes wash-off caused by rain, decomposition by ultra-violet light, oxidation, or hydrolysis in the presence of moisture or, at least such decomposition, oxidation, and hydrolysis as would materially decrease the desirable characteristic of the compound or impart undesirable characteristic for instance, phytotoxicity, to the compound. It will be appreciated that the compounds of this invention can also be used in combination with other biologically active compounds.

Although the invention has been illustrated by the forgoing examples, it is not to be construed

as being limited to the materials employed therein; but rather, the invention encompasses the generic area as hereinafter disclosed.

CLAIMS:

1. A compound of the general formula:

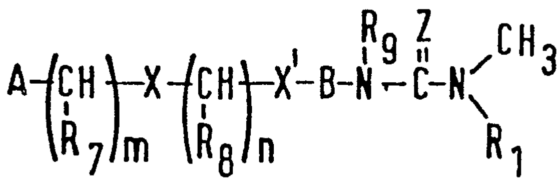

wherein:

A is

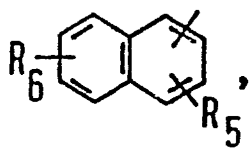, 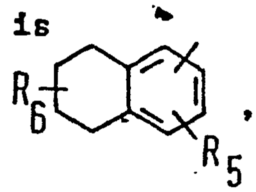, 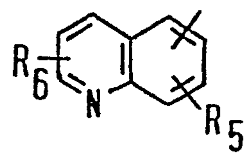, 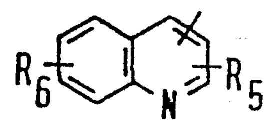,

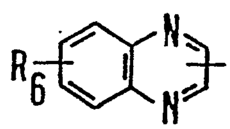, 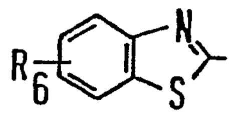 or 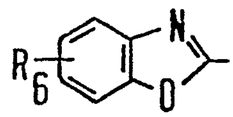

B is

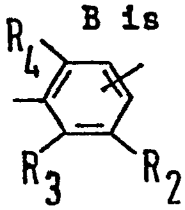 or 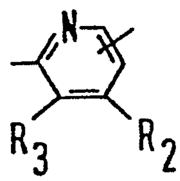

X and X' are independently oxygen, sulfur, or a carbon-carbon, carbon-oxygen, or a carbon-sulfur single bond with the proviso that X and X' may not both be a carbon-carbon single bond at the same time;

Z is oxygen or sulfur;

$R_1$ is hydrogen, $C_1$-$C_8$ alkyl, or $C_1$-$C_3$ alkoxy;

$R_2$-$R_6$ are independently hydrogen, $C_1$-$C_{10}$ alkyl, halogen, cyano, nitro, trifluoromethyl, $C_1$-$C_5$ alkoxyl, or substituted phenoxy; $R_6$ may also be keto when attached to a saturated ring;

$R_7$-$R_9$ are independently hydrogen or lower ($C_1$-$C_3$) alkyl; and

m and n represent the same or different integers in the range 0 to 10 with the proviso that m and n may not both be zero when A is 2-napthyl.

2. A compound as claimed in claim 1 wherein A is

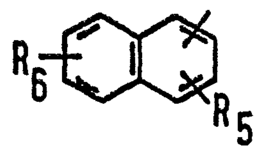

wherein $R_5$ and $R_6$ are as defined in claim 1.

3. A compound as claimed in claim 2 wherein A is a 1-napthyl.

4. A compound as claimed in any of of claims 1 to 3 wherein B is 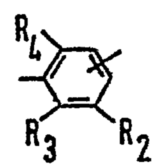

and $R_2$-$R_4$are as defined in claim 1.

5. A compound as claimed in any one of the preceding claims wherein X' and Z are oxygen and m and n are zero.

6. A compound as claimed in any one of claims 1 to 4 wherein m is 1, X is oxygen, n is zero and X' is a C-O bond.

7. A compound as claimed in any one of claims 1 to 4 wherein X and Z are oxygen, m is 1 or 2, n=O, and X' is a C-O bond.

8. A compound as claimed in any one of claims 1 to 4 wherein X' is sulfur, Z is oxygen, m is 2, n is zero, and X is a C-S bond.

9. A compound as claimed in claim 7 when appendant to claim 4 wherein A is

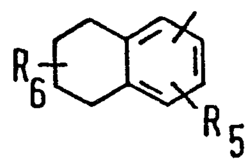

wherein $R_5$ and $R_6$ are as defined in claim 1.

10 . The compound of claim 1 which is 1-methyl-1-methoxy-3-[4-[2-(1-naphth)]ethyloxyphenyl]urea.

11 . The compound of claim 1 which is 1,1-dimethyl-3-[4-[2-(1-naphth)]ethyloxyphenyl]urea.

12 . The compound of claim 1 which is 1-methyl-1-methoxy-3-[4-[2-(1-naphth)]ethyloxy-3-chlorophenyl]urea.

13 . The compound of claim 1 which is 1,1-dimethyl-3-[4-[2-(1-naphth)]ethyloxy-3-chlorophenyl]urea.

14. The compound of claim 1 which is 1-methyl-1-methoxy-3-[4-(5,6,7,8-tetrahydro-1-napthoxy)phenyl]urea.

15 . The compound of claim 1 which is 1,1-dimethyl-3-[4-(5,6,7,8-tetrahydro-1-naphthoxy)phenyl]urea.

16 . The compound of claim 1 which is 1-methyl-1-methoxy-3-[4-(5,6,7,8-tetrahydro-1-naphthoxy)-3-chlorophenyl]urea.

17. The compound of claim 1 which is 1,1-dimethyl-3-[4-(5,6,7,8-tetrahydro-1-naphthoxy)-3-chlorophenyl]urea.

18. The compound of claim 1 which is 1-methyl-1-methoxy-3-[4-(5,6,7,8-tetrahydro-1-naphthoxy)-3-chlorophenyl]urea.

19. The compound of claim 1 which is 1-methyl-1-methoxy-3[4-(5,6,7,8-tetrahydro-2-naphthoxy)-3-chlorophenyl]urea.

20. The compound of claim 1 which is 1-methyl-1-methoxy-3-[4-(5,6,7,8-tetrahydro-2-naphthoxy)-phenyl]urea.

21. A herbicidal composition comprising an acceptable carrier and a herbicidally effective amount of a compound as claimed in any one of the preceding claims.

22. A method for the selective control of undesirable vegetation which comprises applying to the locus to be treated a herbicidally effective amount of a composition as claimed in claim 21.

23. A process for preparing a compound as claimed in claim 1 which comprises reacting a compound of the formula:-

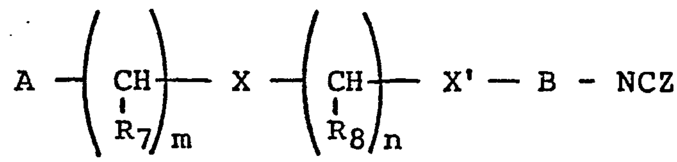

with an aniline of the formula:

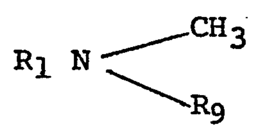

wherein A, B, X, X', Z, $R_1$ -$R_9$, m, and n are as defined in claim 1.

24. A process for the preparation of a compound of the general formula:

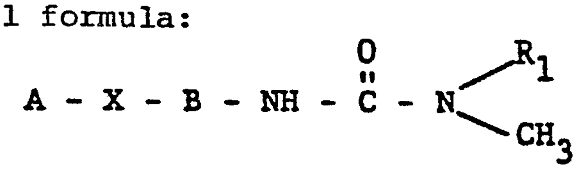

which comprises reacting an aryl isocyanate isocyanate of the formula:

A - X - B - NCO

with an aniline of the formula:

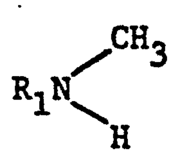

wherein in each occurrence A, X, B and $R_1$ are as defined in claim 1.

25. A process for the preparation of a compound of the general formula:

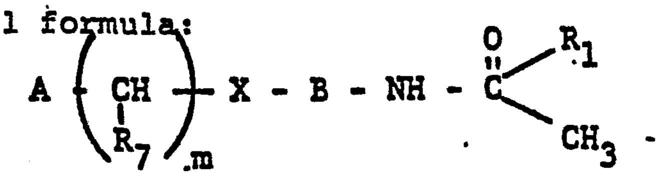

which comprises reacting an aralkyl isocyanate of the formula:

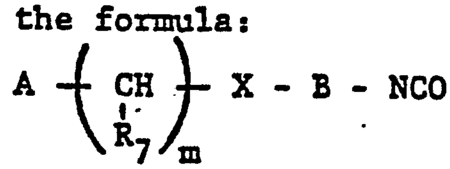

with an aniline of the formula:

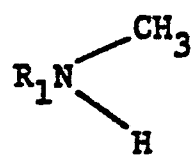

wherein in each occurrence A, X, B, $R_1$ and m are as defined in claim 1.